(19) Europäisches Patentamt
European Patent Office
Office européen des brevets

(11) **EP 4 769 418 A1**

(12) **EUROPEAN PATENT APPLICATION**
published in accordance with Art. 153(4) EPC

(43) Date of publication:
**01.07.2026 Bulletin 2026/27**

(21) Application number: **24885720.3**

(22) Date of filing: **29.10.2024**

(51) International Patent Classification (IPC):
**G16C 10/00** (2019.01)   **G16C 20/00** (2019.01)

(52) Cooperative Patent Classification (CPC):
**G16C 10/00; G16C 20/00**

(86) International application number:
**PCT/JP2024/038563**

(87) International publication number:
**WO 2025/094953 (08.05.2025 Gazette 2025/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.10.2023 US 202363594047 P**

(71) Applicant: **Chugai Seiyaku Kabushiki Kaisha
Kita-ku
Tokyo 115-8543 (JP)**

(72) Inventors:
• **ARAKAWA Akihiko**
  **Yokohama City, Kanagawa 244-8602 (JP)**
• **BRUBAKER Kyle**
  **Seattle, WA 98109-5210 (US)**
• **BOOTH Kyle**
  **Seattle, WA 98109-5210 (US)**
• **FURRER Fabian**
  **Seattle, WA 98109-5210 (US)**
• **GHOSH Jayeeta**
  **Seattle, WA 98109-5210 (US)**

(74) Representative: **Vossius & Partner
Patentanwälte Rechtsanwälte mbB
Siebertstraße 3
81675 München (DE)**

(54) **ESTIMATION SYSTEM, METHOD FOR ESTIMATING MOLECULAR CONFORMATION, AND ESTIMATION PROGRAM**

(57)    An estimation system comprises at least one processor. The at least one processor acquires a target molecule model representing a conformation of a target molecule, generates a molecule-of-interest model representing a conformation of a molecule of interest, the molecule of interest being a molecule capable of binding to the target molecule, calculates energy of interaction between the molecule of interest and the target molecule as intermolecular interaction energy based on a positional relationship between the target molecule model and the molecule-of-interest model, calculates energy of interaction in the molecule of interest as intramolecular interaction energy, and estimates a conformation of the molecule of interest based on the intermolecular interaction energy and the intramolecular interaction energy.

**Fig.1**

## Description

[Technical Field]

An aspect of the present disclosure relates to an estimation system, a method for estimating a molecular conformation, and an estimation program.

[Background Art]

Patent Literature 1 discloses a method for searching for a structure of a cyclic molecule using a computer, comprising searching for a stable structure of a cyclic molecule in which n compound groups are linked in a cyclic form. Patent Literature 2 discloses a device for searching for a stable binding structure of a molecule. Patent Literature 3 discloses a structure searching device that searches for a stable structure of a plurality of molecules that have interactions with each other. Non Patent Literature 1 discloses a method for estimating a conformation of a polymer chain having N monomers on a lattice using a variational quantum algorithm.

[Citation List]

[Patent Literature]

[Patent Literature 1] Japanese Patent Laid-Open No. 2020-91518
[Patent Literature 2] Japanese Patent Laid-Open No. 2020-173643
[Patent Literature 3] Japanese Patent Laid-Open No. 2020-194487

[Non Patent Literature]

[Non Patent Literature 1] Robert, A., Barkoutsos, P.K., Woerner, S. & Tavernelli, I. Resource-efficient quantum algorithm for protein folding. npj Quantum Inf. 7, 38 (2021).

[Summary of Invention]

[Technical Problem]

It is desirable to more accurately estimate a conformation of a molecule capable of binding to a target molecule. An object of an aspect of the present disclosure is to provide an estimation system, a method for estimating a molecular conformation, and an estimation program.

[Solution to Problem]

An estimation system according to an aspect of the present disclosure comprises at least one processor. The at least one processor acquires a target molecule model representing a conformation of a target molecule, generates a molecule-of-interest model representing a conformation of a molecule of interest, the molecule of interest being a molecule capable of binding to the target molecule, calculates energy of interaction between the molecule of interest and the target molecule as intermolecular interaction energy based on a positional relationship between the target molecule model and the molecule-of-interest model, calculates energy of interaction in the molecule of interest as intramolecular interaction energy, and estimates a conformation of the molecule of interest based on the intermolecular interaction energy and the intramolecular interaction energy.

A method for estimating a molecular conformation according to an aspect of the present disclosure is executed by an estimation system comprising at least one processor. The estimation method comprises the steps of: acquiring a target molecule model representing a conformation of a target molecule; generating a molecule-of-interest model representing a conformation of a molecule of interest, the molecule of interest being a molecule capable of binding to the target molecule; calculating energy of interaction between the molecule of interest and the target molecule as intermolecular interaction energy based on a positional relationship between the target molecule model and the molecule-of-interest model; calculating energy of interaction in the molecule of interest as intramolecular interaction energy; and estimating a conformation of the molecule of interest based on the intermolecular interaction energy and the intramolecular interaction energy.

An estimation program according to an aspect of the present disclosure causes a computer to execute the steps of: acquiring a target molecule model representing a conformation of a target molecule; generating a molecule-of-interest model representing a conformation of a molecule of interest, the molecule of interest being a molecule capable of binding to the target molecule; calculating energy of interaction between the molecule of interest and the target molecule as intermolecular interaction energy based on a positional relationship between the target molecule model and the molecule-of-interest model; calculating energy of interaction in the molecule of interest as intramolecular interaction energy; and estimating a conformation of the molecule of interest based on the intermolecular interaction energy and the intramolecular interaction energy.

In such aspects, the energy of interaction between the two molecules is calculated based on the positional relationship between a target molecule and a molecule of interest that are bound to each other. In addition, energy of interaction in the molecule of interest is also calculated. The conformation of the molecule of interest is estimated based on the two types of interaction energy. By considering these two types of interaction energy, the conformation of a molecule of interest which is capable of binding to a target molecule can be more accurately estimated.

[Advantageous Effect of Invention]

According to an aspect of the present disclosure, a conformation of a molecule capable of binding to a target molecule can be more accurately estimated.

[Brief Description of Drawings]

[Figure 1] Figure 1 shows a diagram illustrating an example of a functional configuration of an estimation system.
[Figure 2] Figure 2 shows a diagram illustrating an example of a hardware configuration of a computer that functions as an estimation system.
[Figure 3] Figure 3 shows a flowchart illustrating an example of a process executed by an estimation system.
[Figure 4] Figure 4 shows a diagram illustrating an example of a lattice system.
[Figure 5] Figure 5 shows a diagram illustrating an example in which a molecule-of-interest model is generated under a constraint condition.
[Figure 6] Figure 6 shows a diagram illustrating another example in which a molecule-of-interest model is generated under a constraint condition.
[Figure 7] Figure 7 shows a diagram illustrating the results of Examples.
[Figure 8] Figure 8 shows a diagram illustrating the results of Examples.
[Figure 9] Figure 9 shows a diagram illustrating the results of Examples.
[Figure 10] Figure 10 shows a diagram illustrating the results of Comparative Example.

[Description of Embodiments]

Hereinafter, various examples of the present disclosure will be described in detail with reference to the accompanying drawings. In description of the drawings, identical or equivalent elements are provided with the same reference signs, and redundant description thereof is omitted.

[Outline of system]

An estimation system according to the present disclosure is a computer system for estimating a conformation of a molecule of interest which is a molecule capable of binding to a target molecule. The estimation system processes a conformation model which is electronic data showing a molecular conformation. In the present disclosure, the conformation model of a target molecule is referred to as a "target molecule model", and the conformation model of a molecule of interest is referred to as a "molecule-of-interest model". The estimation system calculates energy of interaction between the molecule of interest and the target molecule as intermolecular interaction energy based on the target molecule model and the molecule-of-interest model. The estimation system also calculates energy of interaction within the molecule of interest as intramolecular interaction energy based on the molecule-of-interest model. The estimation system then estimates a conformation of the molecule of interest based on the intermolecular interaction energy and the intramolecular interaction energy.

In an example, the estimation system generates a molecule-of-interest model for each of a plurality of conformations of a molecule of interest, and calculates intermolecular interaction energy and intramolecular interaction energy for the molecule-of-interest model. The estimation system then selects at least one conformation from a plurality of conformations of the molecule of interest based on the intermolecular interaction energy and the intramolecular interaction energy which are obtained for each of a plurality of molecule-of-interest models, and outputs the selected conformation as an estimation result.

[Configuration of system]

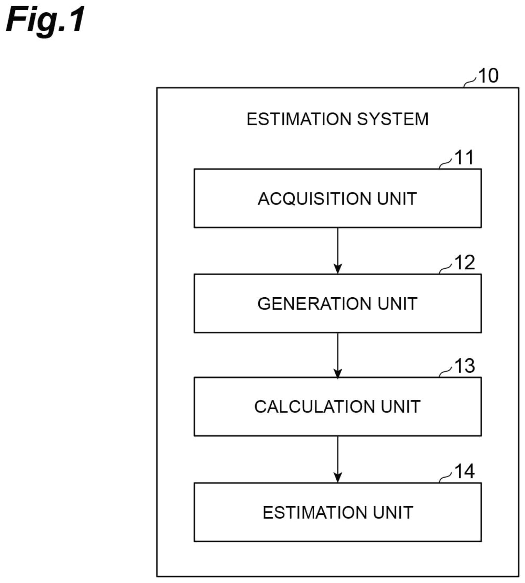

Figure 1 shows a functional configuration of an estimation system 10 according to an example. In this example, the estimation system 10 comprises an acquisition unit 11, a generation unit 12, a calculation unit 13, and an estimation unit 14 as functional modules. The acquisition unit 11 is a functional module that acquires data necessary for estimating a conformation of a molecule of interest. In an example, the acquisition unit 11 acquires a target molecule model, and molecule-of-interest data for use in generation of a molecule-of-interest model. The generation unit 12 is a functional module that generates at least one molecule-of-interest model. The calculation unit 13 is a functional module that, for each of the at least one molecule-of-interest model, calculates intermolecular interaction energy based on a positional relationship between the molecule-of-interest model and a target molecule model, and calculates intramolecular interaction energy based on the molecule-of-interest model. The estimation unit 14 is a functional module that estimates a conformation of the molecule of interest based on intermolecular interaction energy and intramolecular interaction energy for the at least one molecule-of-interest model.

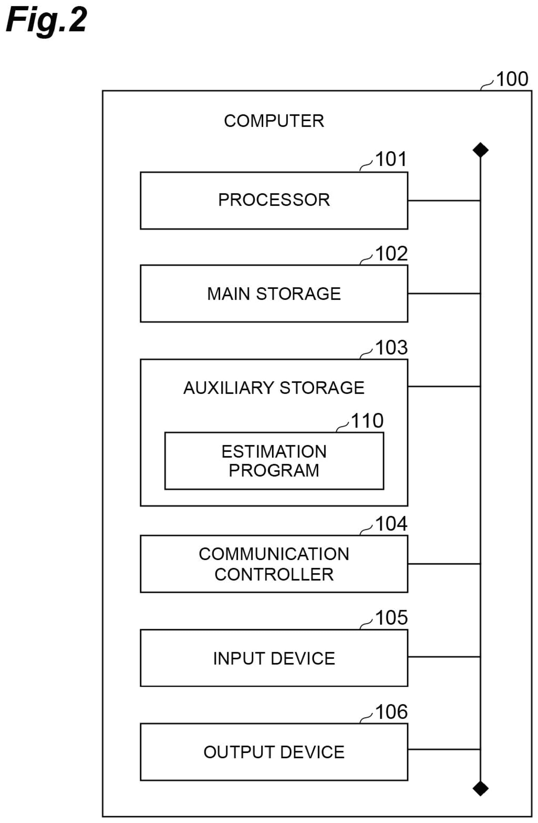

Figure 2 shows a diagram illustrating an example of a hardware configuration of a computer 100 that functions as the estimation system 10. For example, the computer 100 comprises a processor 101, a main storage 102, an auxiliary storage 103, a communication controller 104, an input device 105, and an output device 106. The processor 101 executes an operating system and application programs. The main storage 102 comprises, for example, ROM and RAM. The auxiliary storage 103 comprises a hard disk or a flash memory, and typically stores a larger amount of data than the main storage 102. The communication controller 104 comprises, for example, a network card or a wireless communication module. The input device 105 comprises, for example, a keyboard, a mouse, and a touch panel. The output device 106 comprises, for example, a monitor and a speaker.

The functional modules of the estimation system 10 are implemented by an estimation program 110 stored in advance in the auxiliary storage 103. Specifically, the functional modules are implemented by reading the estimation program 110 into the processor 101 or the main storage 102, and causing the processor 101 to execute the estimation program 110. The processor 101 operates the communication controller 104, the input device 105 or the output device 106 in accordance with the estimation program 110, and reads data from and writes data to the main storage 102 or the auxiliary storage 103. Data or a database necessary for the processing may be stored in the main storage 102 or the auxiliary storage 103.

The estimation program 110 may provided recorded on a non-transitory computer-readable storage medium such as a CD-ROM, a DVD-ROM or a semiconductor memory. Alternatively, the estimation program 110 may be provided via a communication network as a data signal superimposed on a carrier wave.

The estimation system 10 may comprise one computer 100, or may comprise a plurality of computers 100. When a plurality of computers 100 are used, the computers 100 are connected via a communication network such as the internet or an intranet to logically construct one estimation system 10.

[Operation of system]

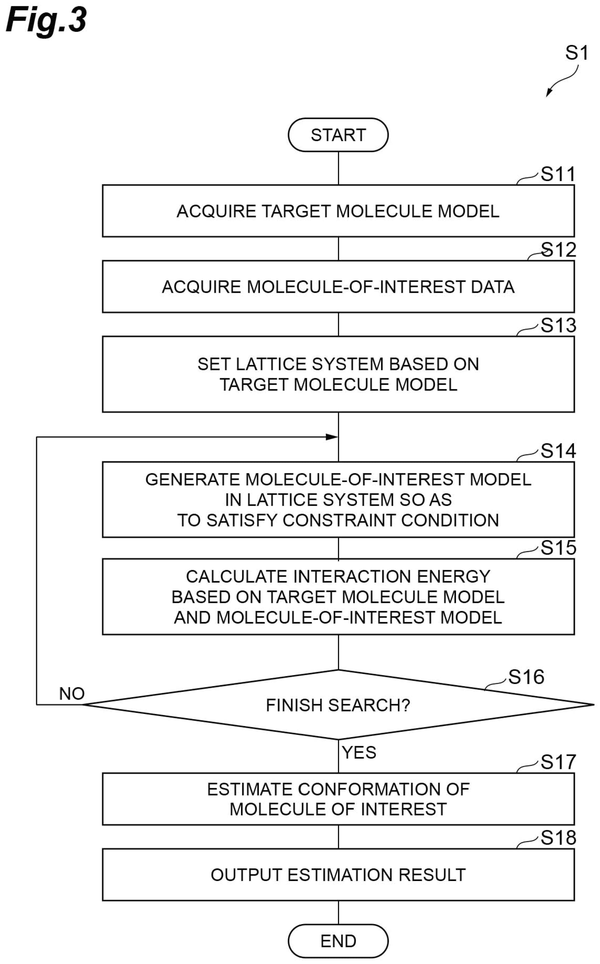

Figure 3 shows a flowchart illustrating an example of a process executed by the estimation system 10, as a process flow S1. The process flow S1 is an example of a method for estimating a molecular conformation according to the present disclosure.

In step S11, the acquisition unit 11 acquires a target molecule model. The target molecule model can be a conformation model specified through input operation or selection operation by a user of the estimation system 10. The acquisition unit 11 may read a target molecule model from a predetermined storage device such as a database, or may receive a target molecule model from another computer such as a user terminal.

In an example, the acquisition unit 11 acquires a target molecule model representing at least one of a plurality of constituent units (groups of atoms) forming a target molecule by a coarse-grained particle. For example, the target molecule model represents each of a plurality of constituent units by a coarse-grained particle. The coarse-graining is a technique in which each constituent unit (group of atoms) forming a system composed of a large number of atoms is approximated as one or more particles each referred to as a coarse-grained particle. The coarse-graining simplifies the constituent unit. Individual constituent units forming a target molecule may be represented by one or more coarse-grained particles. When one constituent unit is represented by two coarse-grained particles, one coarse-grained particle may represent a main chain, with the other coarse-grained particle representing a side chain. The target molecule model may comprise both a constituent unit represented by one coarse-grained particle and a constituent unit represented by two or more coarse-grained particles.

In an example, the acquisition unit 11 may acquire a target molecule model representing a conformation of a part of a target molecule, for example, a target molecule model representing a conformation of an active site in a target molecule. Alternatively, the acquisition unit 11 may acquire a target molecule model representing a conformation of the entire target molecule including an active site.

In step S12, the acquisition unit 11 acquires molecule-of-interest data used for generating a molecule-of-interest model. For example, the molecule-of-interest data is electronic data showing a sequence of a plurality of constituent units forming a molecule of interest. In an example, the molecule-of-interest data is specified through an input operation or a selection operation by a user. The acquisition unit 11 may read molecule-of-interest data from a predetermined storage device such as a database, or may receive molecule-of-interest data from another computer such as a user terminal.

In step S13, the generation unit 12 sets a lattice system based on a target molecule model. The lattice system is a two-dimensional space or a three-dimensional space in which a plurality of lattice points are connected to each other. The lattice system is used for setting a conformation of a molecule of interest. The generation unit 12 sets an outer edge of a lattice system based on a target molecule model. In an example, the generation unit 12 may calculate a center of gravity of a plurality of constituent units forming an active site in a target molecule based on a target molecule model, and set an outer edge of a lattice system based on the center of gravity. For example, the generation unit 12 may set the outer edge of the lattice system at a predetermined distance from the calculated center of gravity. The generation unit 12 arranges a plurality of unit cells inside the outer edge to generate a lattice system. The generation unit 12 may use one of a regular tetrahedral lattice, a cubic lattice, and a square lattice as a unit cell. When a coordinate value of each lattice point of a lattice system is represented as an integer, the generation unit 12 may rearrange the coarse-grained particles of a target molecule model by a technique such as scaling in order to use such a lattice system. In an example, before setting a lattice system, the generation unit 12 may perform at least one of translation and rotational transfer on at least one of a target molecule model and a molecule-of-interest model represented by molecule-of-interest data, thereby rearranging the at least one model.

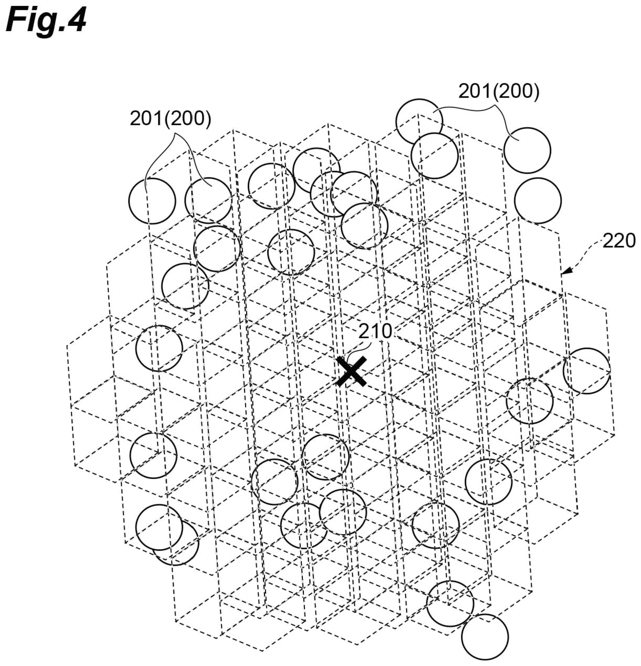

Figure 4 shows a diagram illustrating an example of a lattice system. A target molecule model 200 of this example represents each of a plurality of constituent units forming an active site of a target molecule by a coarse-grained particle 201. The generation unit 12 calculates a center of gravity 210 of a plurality of coarse-grained particles (constituent units) 201, and sets an outer edge of a lattice system 220 at a predetermined distance from the center of gravity. The generation unit 12 arranges unit cells inside the outer edge to generate the lattice system 220.

Returning to Figure 3, in step S14, the generation unit 12 generates a molecule-of-interest model in a lattice system based on molecule-of-interest data such that the molecule-of-interest model satisfies a constraint condition in the lattice system. The generation unit 12 generates the molecule-of-interest model so as to reflect a sequence of a plurality of constituent units forming a molecule of interest. Where j is an integer of 1 or more, the generation unit 12 arranges a j-th constituent unit and a (j+1)-th constituent unit forming a molecule of interest such that the constituent units are adjacent to each other, thereby generating a molecule-of-interest model reflecting the sequence of the molecule. In the present disclosure, the phrase "two constituent units (or two coarse-grained particles) are "adjacent to each other"" means that one constituent unit (or coarse-grained particle) and the other constituent unit (coarse-grained particle) within a predetermined distance from each other. The predetermined distance may be 3.8 Å.

In an example, the generation unit 12 generates a molecule-of-interest model that represents at least one of a plurality of constituent units (groups of atoms) forming a molecule of interest by a coarse-grained particle as in the target molecule model. For example, the generation unit 12 generates a molecule-of-interest model representing each of a plurality of constituent units by a coarse-grained particle. Where j is an integer of 1 or more, the generation unit 12 generates a molecule-of-interest model representing a j-th constituent unit and a (j+1)-th constituent unit forming a molecule of interest by a coarse-grained particle. The generation unit 12 represents (approximates) a constituent unit as one or more coarse-grained particles. The generation unit 12 may represent one constituent unit by two coarse-grained particles. For example, the generation unit 12 may represent the constituent unit by a coarse-grained particle representing a main chain and a coarse-grained particle representing a side chain. In this example, the generation unit 12 arranges the coarse-grained particle representing a main chain and the coarse-grained particle representing a side chain such that the coarse-grained particles are adjacent to each other. The molecule-of-interest model may comprise both a constituent unit coarse-grained by one particle and a constituent unit coarse-grained by two or more particles.

The generation unit 12 may generate a molecule-of-interest model representing at least half or all of the plurality of constituent units forming a molecule of interest by a plurality of coarse-grained particles. For example, the generation unit 12 generates a molecule-of-interest model representing at least half or all of the plurality of constituent units forming a molecule of interest by two coarse-grained particles. The generation unit 12 may generate a molecule-of-interest model representing at least half or all of the plurality of constituent units forming a molecule of interest by a coarse-grained particle representing a main chain and a coarse-grained particle representing a side chain. In an example, the generation unit 12 generates a molecule-of-interest model in the lattice system by arranging a coarse-grained particle representing a main chain and a coarse-grained particle representing a side chain such that the coarse-grained particles are adjacent to each other for at least half or all of the plurality of constituent units forming a molecule of interest.

The generation unit 12 arranges the coarse-grained particles of a molecule-of-interest model at lattice points such that only one coarse-grained particle is arranged at one lattice point. Where j is an integer of 1 or more, the generation unit 12 generates a molecule-of-interest model in the lattice system by arranging two coarse-grained particles corresponding to a j-th constituent unit and a (j+1)-th constituent unit forming a molecule of interest such that the coarse-grained particles are adjacent to each other in the lattice system. In this case, the coarse-grained particle representing the (j+1)-th constituent unit is arranged at a lattice point $L_b$ next to a lattice point $L_a$ at which the coarse-grained particle representing the j-th constituent unit is arranged. When a constituent unit is represented by a coarse-grained particle representing a main chain and a coarse-grained particle representing a side chain, the generation unit 12 generates a molecule-of-interest model in the lattice system by arranging the coarse-grained particle representing a main chain and the coarse-grained particle representing a side chain such that the coarse-grained particles are adjacent to each other. In this case, the coarse-grained particle representing a side chain is arranged at a lattice point $L_d$ next to a lattice point $L_c$ at which the coarse-grained particle representing a main chain is arranged.

The constraint condition is a condition that should be satisfied in generation of a molecule-of-interest model. In an example, the constraint condition includes an intermolecular constraint regarding a positional relationship between a molecule-of-interest model and a target molecule model. For example, the intermolecular constraint shows a requirement that steric hindrance be avoided between a molecule of interest and a target molecule, that is, a requirement that a coarse-grained particle of a molecule-of-interest model be kept at a predetermined distance or longer from a coarse-grained particle of a target molecule model. The constraint condition may include an intramolecular constraint regarding a positional relationship among a plurality of constituent units forming a molecule-of-interest model. For example, the intramolecular constraint shows a requirement that steric hindrance be avoided among a plurality of constituent units forming a molecule of interest, that is, a requirement that coarse-grained particles should not collide with each other in a molecule-of-interest model. The intramolecular constraint may be that a plurality of coarse-grained particles of a molecule of interest be kept at a predetermined distance or longer from one another, or that a plurality of coarse-grained particles of a molecule of interest should not be arranged at one lattice point. The constraint condition may include a cyclization constraint showing a requirement that a molecule of interest contain a cyclic structure, that is, three or more coarse-grained particles be bound in a cyclic form in a molecule-of-interest model. In an example, the constraint condition includes the intermolecular constraint, and at least one of the intramolecular constraint and cyclization constraint.

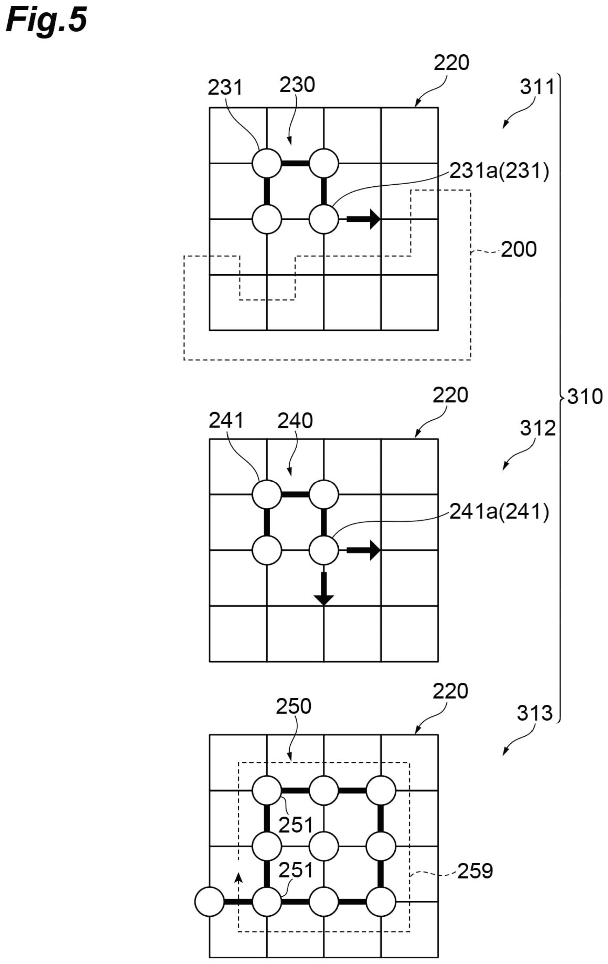

Figure 5 shows a diagram illustrating an example in which a molecule-of-interest model is generated under a constraint condition. In Figure 5, for convenience, a lattice system 220 is shown in a simple two-dimensional shape where a unit cell has a square shape. This example is based on the premise that individual constituent units of a molecule-of-interest model are each represented by one coarse-grained particle. A constraint condition 310 shown in the example includes an intermolecular constraint 311, an intramolecular constraint 312, and a cyclization constraint 313. In the example, these three constraints are described separately for convenience, but it is to be noted that the generation unit 12 generates a molecule-of-interest model so as to satisfy the whole of the constraint condition 310 in the lattice system.

The generation unit 12 generates a molecule-of-interest model 230 in the lattice system 220 based on the intermolecular constraint 311 such that steric hindrance does not occur between a coarse-grained particle 231 of the molecule-of-interest model 230 and a coarse-grained particle of the target molecule model 200. After arrangement of a fourth coarse-grained particle 231a, the generation unit 12 arranges a fifth coarse-grained particle to the right of the coarse-grained particle 231a such that steric hindrance does not occur between the coarse-grained particle and a coarse-grained particle of the target molecule model 200.

The generation unit 12 generates a molecule-of-interest model 240 in the lattice system 220 based on the intramolecular constraint 312 such that steric hindrance does not occur between coarse-grained particles 241 of the molecule-of-interest model 240. After arrangement of a fourth coarse-grained particle 241a, the generation unit 12 arranges a fifth coarse-grained particle to the right or below of the coarse-grained particle 241a such that steric hindrance does not occur between the coarse-grained particle and another coarse-grained particle 241.

The generation unit 12 generates a molecule-of-interest model 250 in the lattice system 220 based on the cyclization constraint 313 such that a cyclic structure 259 is formed by three or more coarse-grained particles 251.

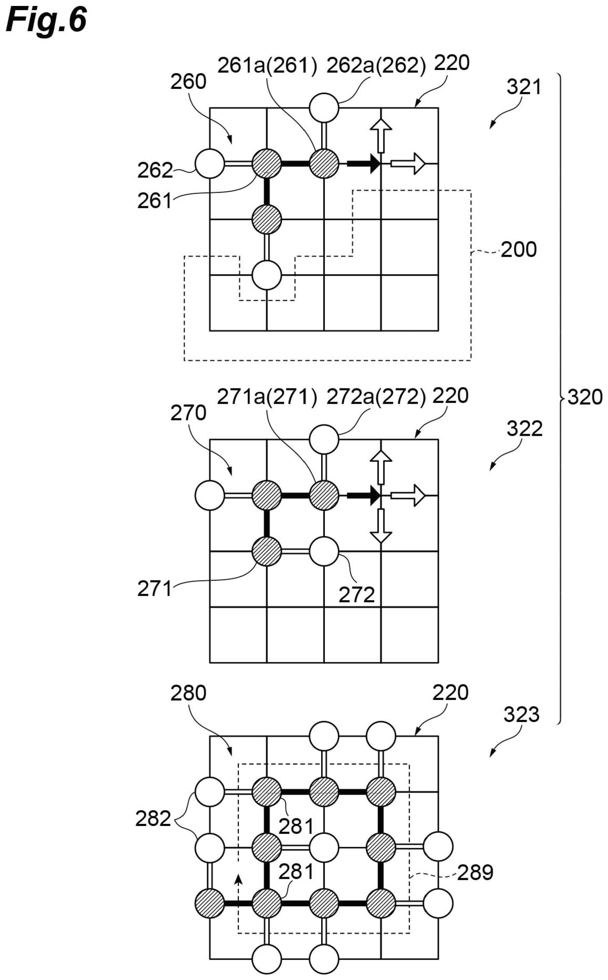

Figure 6 shows another example in which a molecule-of-interest model is generated under a constraint condition. In Figure 6, the lattice system 220 is shown in a simple two-dimensional shape. This example is based on the premise that individual constituent units of a molecule-of-interest model are each represented by two coarse-grained particles. The hatched circle denotes a main chain, and the white circle denotes a side chain. Hereinafter, the coarse-grained particle representing a main chain is also referred to as a "main chain particle", and the coarse-grained particle representing a side chain is also referred to as a "side chain particle". A constraint condition 320 shown in the example includes an intermolecular constraint 321, an intramolecular constraint 322, and a cyclization constraint 323. In the example, these three constraints are described separately for convenience, but it is to be noted that the generation unit 12 generates a molecule-of-interest model so as to satisfy the whole of the constraint condition 320.

The generation unit 12 generates a molecule-of-interest model 260 in the lattice system 220 based on the intermolecular constraint 311 such that steric hindrance does not occur between a main chain particle 261 and a side chain particle 262 of the molecule-of-interest model 260 and a coarse-grained particle of the target molecule model 200. After arrangement of a main chain particle 261a and a side chain particle 262a which represent a third constituent unit, the generation unit 12 arranges a main chain particle and a side chain particle which represent a fourth constituent unit such that steric hindrance does not occur between the particles and a coarse-grained particle of the target molecule model 200. The generation unit 12 arranges the fourth main chain particle to the right of the main chain particle 261a, and arranges the fourth side chain particle on the right of or above the fourth main chain particle.

The generation unit 12 generates a molecule-of-interest model 270 in the lattice system 220 based on the intramolecular constraint 322 such that steric hindrance does not occur between coarse-grained particles of the molecule-of-interest model 270. After arrangement of a main chain particle 271a and a side chain particle 272a which represent a third constituent unit, the generation unit 12 arranges a main chain particle and a side chain particle which represent a fourth constituent unit such that steric hindrance does not occur between these particles and other main chain particles 271 and side chain particles 272. The generation unit 12 arranges the fourth main chain particle to the right of the main chain particle 271a, and arranges the fourth side chain particle to the right of, above or below the fourth main chain particle.

The generation unit 12 generates a molecule-of-interest model 280 in the lattice system 220 based on the cyclization constraint 323 such that a cyclic structure 289 is formed by three or more main chain particles 281. The generation unit 12 arranges a side chain particle 282 next to each of the main chain particles 281.

Returning to Figure 3, in step S15, the calculation unit 13 calculates the interaction energy of the conformation of the molecule of interest based on the target molecule model and the molecule-of-interest model. In an example, the calculation unit 13 calculates intramolecular interaction energy and intermolecular interaction energy, and calculates the sum of the two interaction energies as the total interaction energy of the conformation of the molecule of interest.

Calculation of intramolecular interaction energy will be described. In an example, the calculation unit 13 calculates a plurality of energies of interaction between a plurality of constituent units forming a molecule of interest, as a plurality of energies of interaction between units. The calculation unit 13 calculates intramolecular interaction energy based on a plurality of energies of interaction between units. For example, the calculation unit 13 calculates a sum of a plurality of energies of interaction between units as intramolecular interaction energy. This calculation is represented by equation (1).

[Equation 1]

$$E_{intra} = \sum_{1 \leq i < j \leq N} a_{ij} e_{ij} \quad ...(1)$$

The variable $E_{intra}$ represents intramolecular interaction energy. The variables i and j each represent a number for identifying a constituent unit of a molecule of interest. The variable $a_{ij}$ is a coefficient set based on a distance between the constituent unit i and the constituent unit j. The variable $a_{ij}$ is "1" when the distance is below a predetermined threshold Td, and "0" otherwise. However, when two constituent units are directly bound covalently to each other, the variable $a_{ij}$ is "0" even if the distance is below a predetermined threshold Td. An example of this is a case where the j-th constituent unit and the (j+1)-th constituent unit are bound covalently to each other. The variable $e_{ij}$ represents energy of interaction between units which is generated between constituent units i and j.

Calculation of intermolecular interaction energy will be described. In an example, the calculation unit 13 calculates a plurality of energies of interaction between a plurality of constituent units forming a target molecule and a plurality of constituent units forming a molecule of interest, as a plurality of energies of interaction between units. The calculation unit 13 calculates intermolecular interaction energy based on the plurality of energies of interaction between units. For example, the calculation unit 13 calculates a sum of a plurality of energies of interaction between units as intermolecular interaction energy. This calculation is represented by equation (2).

[Equation 2]

$$E_{inter} = \sum_{i} \sum_{k} b_{ik} e_{ik} \quad ...(2)$$

The variable $E_{inter}$ represents intermolecular interaction energy. The variable i represents a number for identifying a constituent unit of a molecule of interest, and the variable k represents a number for identifying a constituent unit of a target molecule. The variable $b_{ik}$ is a coefficient set based on a distance between the constituent unit i and the constituent unit k. The variable $b_{ik}$ is "1" when the distance is below a predetermined threshold Td, and "0" otherwise. The variable $e_{ik}$ represents energy of interaction between units which is generated between two constituent units i and k.

Equations (1) and (2) are both represent calculating a sum of energies of interaction between two constituent units whose distance from each other is below the threshold Td, that is, between two constituent units that are positioned close to each other. When the constituent units of the molecule of interest and the target molecule are amino acids, the calculation unit 13 may determine the energy of interaction between units and the threshold Td based on the Miyazawa-Jernigan matrix (MJ matrix) (J. Mol. Biol. (1996) 256, 623-644, Table 3) for both equations (1) and (2). The MJ matrix defines energy of interaction between two amino acids. The distance that is referred to for determining the values of variables of $a_{ij}$ and $b_{ik}$ in equations (1) and (2) may be a Euclidean distance. The threshold Td may be set based on 6.5 Å ($6.5 \times 10^{-10}$ m).

As shown in equation (3), the calculation unit 13 calculates the sum of intramolecular interaction energy $E_{intra}$ and intermolecular interaction energy $E_{inter}$ as total interaction energy $E_{total}$.

[Equation 3]

$$E_{total} = E_{intra} + E_{inter} \quad \cdots (3)$$

In an example, as shown in step S16, the generation unit 12 and the calculation unit 13 collaborate to perform a search comprising repeating generation of a molecule-of-interest model and calculation of interaction energy for a plurality of conformations of a molecule of interest. When the process is to be carried out for another conformation (NO in step S16), the process returns to step S14. The generation unit 12 generates a new molecule-of-interest model in the existing lattice system (for example, the lattice system 220) based on molecule-of-interest data so that the molecule-of-interest model satisfies a constraint condition in the lattice system. In step S15 that is repeated, the calculation unit 13 calculates interaction energy (for example, total interaction energy) of a new conformation of the molecule of interest based on the target molecule model and the new molecule-of-interest model.

When the search is finished (YES in step S16), the process proceeds to step S17. In step S17, the estimation unit 14 estimates a conformation of the molecule of interest (molecule-of-interest model). In an example, the estimation unit 14 selects at least one conformation from a plurality of conformations based on interaction energy of each of a plurality of conformations of a molecule of interest, and sets the selected conformation as an estimation result. The estimation unit 14 may select conformations based on total interaction energy. For example, the estimation unit 14 may select the conformation whose total interaction energy is the smallest, or at least one conformation in which the total interaction energy is below the threshold Te. The estimation unit 14 may select conformations based on intermolecular interaction energy. For example, the estimation unit 14 may select the conformation whose intermolecular interaction energy is the smallest, or at least one conformation in which the intermolecular interaction energy is below the threshold Ti.

In step S18, the estimation unit 14 outputs an estimation result showing at least one conformation (molecule-of-interest model) estimated. For example, the estimation unit 14 may output electronic data representing individual conformations in a form that allows the conformation to be written by computer graphics (CG) as an estimation result. The estimation unit 14 may output an estimation result that further shows interaction energy of each conformation estimated, for example, an estimation result that further shows at least one of total interaction energy, intermolecular interaction energy and intramolecular interaction energy. The estimation unit 14 may store the estimation result in a storage device such as an auxiliary storage 103, may display a prediction result on a monitor, or may send the prediction result to another computer such as a user terminal.

As shown in process flow S1, the generation unit 12 generates a plurality of molecule-of-interest models (conformations) of the molecule of interest while changing the orientation or the position of the lattice system with respect to the target molecule model. The calculation unit 13 calculates intermolecular interaction energy and intramolecular interaction energy for each molecule-of-interest model (conformation) generated, and calculates the sum of the two interaction energies as the total interaction energy. The estimation unit 14 selects at least one conformation from a plurality of conformations of the molecule of interest based on interaction energy (for example, total interaction energy or intermolecular interaction energy) of each of a plurality of conformations of the molecule of interest.

As described above, in an example, the estimation unit 14 outputs, as an estimation result, the conformation whose total interaction energy is the smallest. This process can be said to be mathematical optimization. In this case, equation (3) can be said to be an objective function, and the total interaction energy $E_{total}$ can be said to be an objective variable. The conformation whose total interaction energy $E_{total}$ is the smallest is a conformation which is expected to be the most stable (that is, the most stable structure).

[Molecule]

The molecule is not particularly limited, and can be appropriately selected according to a purpose. The molecule may be a small molecule (low-molecular compound), a middle molecule (middle-molecular compound), or a macromolecule (macromolecular compound). In the present disclosure, the small molecule or low-molecular compound is a compound having a molecular weight of less than 500 g/mol. In the present disclosure, the middle molecule or middle-molecular compound is a compound having a molecular weight of 500 g/mol or more and less than 10,000 g/mol. In the present disclosure, the macromolecule or macromolecular compound is a compound having a molecular weight of 10,000 g/mol or more.

The molecule may be a biomolecule or a non-biomolecule. The molecule may be a nucleic acid, a peptide, a protein, or an antigen-binding molecule such as an antibody, or a molecule capable of binding to a target molecule (target molecule-binding molecule). The molecule may be a drug candidate molecule. The peptide may be a linear peptide or a cyclic peptide. When the molecule is a peptide, a protein or an antibody, the constituent units of the molecule are amino acids. When the molecule is a nucleic acid, the constituent units of the molecule are nucleosides or nucleotides. In an example, the target molecule may be a protein such as a receptor or an enzyme, and the molecule of interest may be a peptide. In the present disclosure, the protein as a target molecule is also referred to as a "target protein", and the peptide as a molecule of interest is also referred to as a "peptide of interest". The number of amino acid residues contained in the peptide may be, for example, 5 to 30, 7 to 20, 8 to 18, or 9 to 15. The peptide may be linear, branched, or cyclic. When the peptide is a linear peptide or a cyclic peptide, the linear peptide as a molecule of interest is referred to as a "linear peptide of interest", and the cyclic peptide as a molecule of interest is also referred to as a "cyclic peptide of interest".

The "amino acid" includes a natural amino acid, and an unnatural amino acid. The "natural amino acid" is any L-amino acid selected from Gly (glycine), L-Ala (alanine), L-Ser (serine), L-Thr (threonine), L-Val (valine), L-Leu (leucine), L-Ile (isoleucine), L-Phe (phenylalanine), L-Tyr (tyrosine), L-Trp (tryptophan), L-His (histidine), L-Glu (glutamic acid), L-Asp (aspartic acid), L-Gln (glutamine), L-Asn (asparagine), L-Cys (cysteine), L-Met (methionine), L-Lys (lysine), L-Arg (arginine), and L-Pro (proline). The "unnatural amino acid" is an amino acid other than a natural amino acid. Examples of the unnatural amino acid include $\beta$-amino acids, $\gamma$-amino acids, D-amino acids, N-substituted amino acids other than Pro, $\alpha,\alpha$-disubstituted amino acids, and amino acids having a side chain different from those of natural amino acids. The "main chain of an amino acid" is, in the case of an $\alpha$-amino acid, a chain portion composed of an amino group, $\alpha$-carbon and a carboxyl group. In the case of a $\beta$-amino acid, the "main chain of an amino acid" is a chain portion composed of an amino group, $\beta$-carbon, $\alpha$-carbon and a carboxyl group. In the case of a $\gamma$-amino acid, the "main chain of an amino acid" is a chain portion composed of an amino group, $\gamma$-carbon, $\beta$-carbon, $\alpha$-carbon and a carboxyl group. The "side chain of an amino acid" is, in the case of an $\alpha$-amino acid, a group and/or an atom bonded to carbon ($\alpha$-carbon) to which an amino group and a carboxyl group are bonded. For example, the methyl group of Ala is a side chain of the amino acid. On the other hand, Gly (glycine) has no side chain. In the case of a $\beta$-amino acid, a group and/or an atom bonded to at least one of $\alpha$-carbon and $\beta$-carbon form a side chain of the amino acid. In the case of a $\gamma$-amino acid, a group and/or an atom bonded to at least one of $\alpha$-carbon, $\beta$-carbon and $\gamma$-carbon can form a side chain of the amino acid.

[Modifications]

The technique of the present disclosure has been described in detail above based on various examples. However, the technique of the present disclosure is not limited to the above examples. Various modifications can be made without departing from the spirit of the present disclosure.

In the process flow S1, the estimation system 10 generates a plurality of conformations (molecule-of-interest models) of the molecule of interest, and selects at least one conformation from the plurality of conformations. However, the estimation system 10 may calculate intermolecular interaction energy and intramolecular interaction energy for one conformation (molecule-of-interest model) of the molecule of interest, and estimate the conformation of the molecule of interest based on these interaction energies. That is, the repetition shown by step S16 of process flow S1 may be omitted.

The estimation system may be constructed as a server in a client-server system, or implemented in a stand-alone computer. Alternatively, the estimation system may be implemented in a user terminal which allows a predetermined database to be accessed through a communication network such as the Internet.

The procedure of a method executed by at least one processor is not limited to examples in the embodiments described above. For example, a part of the steps or processes described above may be omitted, and the steps may be executed in a different order. Any two or more of the steps described above may be combined, and a part of the steps may be modified or deleted. Alternatively, in addition to the steps described above, other steps may be executed.

In comparison of the magnitudes of two numerical values in the present disclosure, any of two criteria, i.e., "or more" and "more than" criteria may be used, and any of two criteria, i.e., "or less" and "less than" criteria may be used.

In the present disclosure, the wording "at least one processor executes a first process, executes a second process, ..., and executes an nth process", or equivalent wording indicates a concept including a case where the processor to execute n processes from the first to nth processes changes in the middle. That is, this wording indicates a concept including both a case where all n processes are executed by the same processor and a case where the processor is changed in n processes due to any policy.

In the present disclosure, the term "to" that indicates a range includes values of both ends thereof. For example, "A to B" means the range of A or more and B or less.

In the present disclosure, the term "about" when used in combination with a numerical value means a range of +10% and -10% of the numerical value.

In the present specification, the term "and/or" is used to show the matters described before and after "and/or", or any combination thereof. For example, "A, B and/or C" includes the matters "A", "B" and "C", and combinations "A and B", "A and C", "B and C", and "A and B and C".

[Supplements]

As is understood from the various examples described above, the present disclosure includes the following aspects.

(Supplement 1)

An estimation system comprising at least one processor,
wherein the at least one processor

acquires a target molecule model representing a conformation of a target molecule,
generates a molecule-of-interest model representing a conformation of a molecule of interest, the molecule of interest being a molecule capable of binding to the target molecule,
calculates energy of interaction between the molecule of interest and the target molecule as intermolecular interaction energy based on a positional relationship between the target molecule model and the molecule-of-interest model,
calculates energy of interaction in the molecule of interest as intramolecular interaction energy, and
estimates a conformation of the molecule of interest based on the intermolecular interaction energy and the intramolecular interaction energy.

(Supplement 2)

The estimation system according to supplement 1,
wherein the at least one processor

calculates a plurality of energies of interaction between a plurality of constituent units forming the target molecule and a plurality of constituent units forming the molecule of interest, as a plurality of energies of interaction between units, and
calculates the intermolecular interaction energy based on the plurality of energies of interaction between units.

(Supplement 3)

The estimation system according to supplement 1 or 2,
wherein the at least one processor

calculates a plurality of energies of interaction between a plurality of constituent units forming the molecule of interest, as a plurality of energies of interaction between units, and
calculates the intramolecular interaction energy based on the plurality of energies of interaction between units.

(Supplement 4)

The estimation system according to supplement 2 or 3,
wherein the at least one processor

acquires the target molecule model representing the plurality of constituent units forming the target molecule by coarse-grained particles, and
generates the molecule-of-interest model representing the plurality of constituent units forming the molecule of interest by the coarse-grained particles.

(Supplement 5)

The estimation system according to supplement 4,
wherein the at least one processor

arranges a j-th constituent unit and a (j+1)-th constituent unit forming the molecule of interest such that the constituent units are adjacent to each other, with j being an integer of 1 or more, and
generates the molecule-of-interest model representing the j-th constituent unit and the (j+1)-th constituent unit by the coarse-grained particles.

(Supplement 6)

The estimation system according to supplement 4 or 5,
wherein the at least one processor generates the molecule-of-interest model representing at least one of the plurality of constituent units forming the molecule of interest by a plurality of the coarse-grained particles.

(Supplement 7)

The estimation system according to supplement 6,
wherein the at least one processor generates the molecule-of-interest model representing at least one of the plurality of constituent units forming the molecule of interest by two of the coarse-grained particles.

(Supplement 8)

The estimation system according to any one of supplements 4 to 7,
wherein the at least one processor generates the molecule-of-interest model representing at least one of the plurality of constituent units forming the molecule of interest by the coarse-grained particle representing a main chain and the coarse-grained particle representing a side chain.

(Supplement 9)

The estimation system according to supplement 8,
wherein the at least one processor generates the molecule-of-interest model by arranging the coarse-grained particle representing the main chain and the coarse-grained particle representing the side chain such that the coarse-grained particles are adjacent to each other.

(Supplement 10)

The estimation system according to any one of supplements 1 to 9,
wherein the at least one processor

generates the molecule-of-interest model for each of the plurality of conformations of the molecule of interest, calculates the intermolecular interaction energy based on the positional relationship between the target molecule model and the generated molecule-of-interest model, and
selects the at least one conformation from the plurality of conformations of the molecule of interest based on the intermolecular interaction energy of each of the plurality of conformations of the molecule of interest.

(Supplement 11)

The estimation system according to supplement 10,
wherein the at least one processor

generates the molecule-of-interest model for each of the plurality of conformations of the molecule of interest, calculates energy of interaction in the molecule of interest as intramolecular interaction energy based on the generated molecule-of-interest model, calculates the sum of the intramolecular interaction energy and the intermolecular interaction energy as total interaction energy, and
selects at least one conformation from the plurality of conformations of the molecule of interest based on the total interaction energy of each of the plurality of conformations of the molecule of interest.

(Supplement 12)

The estimation system according to any one of supplements 1 to 11,
wherein the at least one processor generates the molecule-of-interest model so as to satisfy a constraint condition including an intermolecular constraint regarding the positional relationship between the molecule-of-interest model and the target molecule model.

(Supplement 13)

The estimation system according to supplement 12,
wherein the intermolecular constraint indicates that steric hindrance is avoided between the molecule of interest and the target molecule.

(Supplement 14)

The estimation system according to supplement 12 or 13,
wherein the at least one processor

sets an outer edge of a lattice system for arranging the conformation of the molecule of interest, based on the target molecule model, and
generates the molecule-of-interest model in the lattice system such that the molecule-of-interest model satisfies the constraint condition in the lattice system.

(Supplement 15)

The estimation system according to supplement 14,
wherein the at least one processor

acquires the target molecule model representing the conformation of an active site in the target molecule, and
sets the outer edge of the lattice system based on a center of gravity of a plurality of constituent units forming the active site.

(Supplement 16)

The estimation system according to supplement 14 or 15,
wherein a unit cell of the lattice system is one selected from a regular tetrahedral lattice, a cubic lattice and a square lattice.

(Supplement 17)

The estimation system according to any one of supplements 14 to 16,
wherein the at least one processor generates the molecule-of-interest model in the lattice system by arranging two coarse-grained particles corresponding to a j-th constituent unit and a (j+1)-th constituent unit forming the molecule of interest such that the coarse-grained particles are adjacent to each other in the lattice system, with j being an integer of 1 or more.

(Supplement 18)

The estimation system according to any one of supplements 14 to 17,
wherein the at least one processor generates the molecule-of-interest model in the lattice system by arranging a coarse-grained particle representing a main chain and a coarse-grained particle representing a side chain such that the coarse-grained particles are adjacent to each other for at least one of a plurality of constituent units forming the molecule of interest in the lattice system.

(Supplement 19)

The estimation system according to any one of supplements 12 to 18,
wherein the constraint condition further includes at least one of an intramolecular constraint indicating that steric hindrance is avoided between a plurality of constituent units forming the molecule of interest and a cyclization constraint indicating that the molecule of interest contains a cyclic structure.

(Supplement 20)

The estimation system according to any one of supplements 1 to 19,

wherein the target molecule is a protein, and
the molecule of interest is a peptide.

(Supplement 21)

A method for estimating a molecular conformation, which is executed by an estimation system comprising at least one processor, the method comprising the steps of:

acquiring a target molecule model representing a conformation of a target molecule;
generating a molecule-of-interest model representing a conformation of a molecule of interest, the molecule of interest being a molecule capable of binding to the target molecule;
calculating energy of interaction between the molecule of interest and the target molecule as intermolecular interaction energy based on a positional relationship between the target molecule model and the molecule-of-interest model;
calculating energy of interaction in the molecule of interest as intramolecular interaction energy; and
estimating a conformation of the molecule of interest based on the intermolecular interaction energy and the intramolecular interaction energy.

(Supplement 22)

An estimation program that causes a computer to execute the steps of:

acquiring a target molecule model representing a conformation of a target molecule;
generating a molecule-of-interest model representing a conformation of a molecule of interest, the molecule of interest being a molecule capable of binding to the target molecule;
calculating energy of interaction between the molecule of interest and the target molecule as intermolecular interaction energy based on a positional relationship between the target molecule model and the molecule-of-interest model;
calculating energy of interaction in the molecule of interest as intramolecular interaction energy; and
estimating a conformation of the molecule of interest based on the intermolecular interaction energy and the intramolecular interaction energy.

(Supplement 23)

The estimation system according to supplement 4 or 5,
wherein the at least one processor generates the molecule-of-interest model representing at least half of the plurality of constituent units forming the molecule of interest by a plurality of the coarse-grained particles.

(Supplement 24)

The estimation system according to supplement 4 or 5,
wherein the at least one processor generates the molecule-of-interest model representing all of the plurality of constituent units forming the molecule of interest by a plurality of the coarse-grained particles.

(Supplement 25)

The estimation system according to supplement 6,
wherein the at least one processor generates the molecule-of-interest model representing at least half of the plurality of constituent units forming the molecule of interest by two of the coarse-grained particles.

(Supplement 26)

The estimation system according to supplement 6,
wherein the at least one processor generates the molecule-of-interest model representing all of the plurality of constituent units forming the molecule of interest by two of the coarse-grained particles.

(Supplement 27)

The estimation system according to any one of supplements 4 to 7,
wherein the at least one processor generates the molecule-of-interest model representing at least half of the plurality of constituent units forming the molecule of interest by the coarse-grained particle representing a main chain and the coarse-grained particle representing a side chain.

(Supplement 28)

The estimation system according to any one of supplements 4 to 7,
wherein the at least one processor generates the molecule-of-interest model representing all of the plurality of constituent units forming the molecule of interest by the coarse-grained particle representing a main chain and the coarse-grained particle representing a side chain.

(Supplement 29)

The estimation system according to any one of supplements 14 to 17,
wherein the at least one processor generates the molecule-of-interest model in the lattice system by arranging a coarse-grained particle representing a main chain and a coarse-grained particle representing a side chain such that the coarse-grained particles are adjacent to each other for at least half of a plurality of constituent units forming the molecule of interest in the lattice system.

(Supplement 30)

The estimation system according to any one of supplements 14 to 17,
wherein the at least one processor generates the molecule-of-interest model in the lattice system by arranging a coarse-grained particle representing a main chain and a coarse-grained particle representing a side chain such that the coarse-grained particles are adjacent to each other for all of the plurality of constituent units forming the molecule of interest in the lattice system.

According to supplements 1, 21 and 22, energy of interaction between the two molecules is calculated based on a positional relationship between a target molecule and a molecule of interest which are bound to each other. In addition, energy of interaction in the molecule of interest is calculated. The conformation of the molecule of interest is estimated based on the two types of interaction energy. By considering these two types of interaction energy, the conformation of a molecule of interest which is capable of binding to a target molecule can be more accurately estimated. By accurately estimating the conformation, the binding mode of the molecule of interest to the target molecule can be estimated.

According to supplement 2, intermolecular interaction energy is calculated based on energy of interaction between the constituent unit of the target molecule and the constituent unit of the molecule of interest, so that more accurate intermolecular interaction energy can be obtained. By this accurate calculation, the accuracy of estimating the conformation of the molecule of interest can be further improved.

According to supplement 3, intramolecular interaction energy is calculated based on energy of interaction between the constituent units of the molecule of interest, so that more accurate intramolecular interaction energy can be obtained. By this accurate calculation, the accuracy of estimating the conformation of the molecule of interest can be further improved.

According to supplement 4, the constituent units are represented by a coarse-grained particle for both the target molecule and the molecule of interest. Since the coarse-graining simplifies the target molecule model and the molecule-of-interest model, the calculation of interaction energy is simplified, so that the the calculation time can be reduced. As a result, the conformation of the molecule of interest can be estimated more rapidly.

According to supplement 5, a molecule-of-interest model using coarse-grained particles is generated so as to reflect the arrangement of constituent units of the molecule of interest. By using a molecule-of-interest model that more accurately reflects the structure of the molecule of interest, the accuracy of estimating the conformation of the molecule of interest can be further improved.

According to supplement 6, at least a part of a plurality of constituent units of the molecule of interest is represented by a plurality of coarse-grained particles, so that a molecule-of-interest model representing the structure of the molecule of interest more precisely is generated. By using the molecule-of-interest model, the conformation of the molecule of interest can be more precisely estimated.

According to supplement 7, at least a part of a plurality of constituent units of the molecule of interest is represented by two coarse-grained particles. By using the molecule-of-interest model, a balance can be achieved between the reduction of the time of calculation of interaction energy and precise estimation of the conformation.

According to supplement 8, at least a part of a plurality of constituent units of the molecule of interest is represented by two coarse-grained particles representing a main chain and a side chain, so that a molecule-of-interest model representing the structure or the function of the constituent unit is generated. By using the molecule-of-interest model, the conformation of the molecule of interest can be estimated more precisely and more accurately.

According to supplement 9, a molecule-of-interest model using coarse-grained particles is generated so as to reflect the arrangement of main chains and side chains. By using a molecule-of-interest model that more accurately reflects the structure of the molecule of interest, the accuracy of estimating the conformation of the molecule of interest can be further improved.

According to supplement 10, a plurality of molecule-of- interest models (conformations) are generated for a certain molecule of interest, and at least one conformation is selected based on the intermolecular interaction energy of each molecule-of-interest model. By the search taking intermolecular interaction energy into consideration, a highly plausible conformation can be more accurately estimated from a plurality of conformation candidates for the molecule of interest.

According to supplement 11, a plurality of molecule-of- interest models (conformations) are generated for a molecule of interest, and at least one conformation is selected based on the total interaction energy of each molecule-of-interest model. By the search taking both intermolecular interaction energy and intramolecular interaction energy into consideration, a highly plausible conformation can be more accurately estimated from a plurality of conformation candidates for the molecule of interest.

According to supplement 12, the molecule-of-interest model is generated so as to satisfy an intermolecular constraint regarding the positional relationship between the molecule-of-interest model and the target molecule model, so that a molecule-of-interest modelconsistent with the behaviors of the molecule of interest and the target molecule in the real world is obtained. By using the molecule-of-interest model, the conformation of the molecule of interest can be more accurately estimated.

According to supplement 13, the molecule-of-interest model is generated so as to avoid steric hindrance between the molecule of interest and the target molecule, so that an appropriate molecule-of-interest model that is free from molecular strain and collision between the molecules is obtained. By using the molecule-of-interest model, the conformation of the molecule of interest can be more accurately estimated.

According to supplement 14, in a lattice system set based on a target molecule model, a molecule-of-interest model is generated by considering constraint conditions. By introducing the lattice system, generation of the molecule-of-interest model is simplified, so that the molecule-of-interest model can be generated rapidly.

According to supplement 15, an outer edge of the lattice system is set based on the center of gravity of an active site of the target molecule. By defining the range of the lattice system as described above, a molecule-of-interest model consistent with the behaviors of the molecule of interest and the target molecule in the real world can be efficiently generated.

According to supplement 16, by setting the unit cell of the lattice system to a regular tetrahedral lattice, a cubic lattice or a square lattice, a molecule-of-interest model can be accurately generated while generation of the molecule-of-interest model is simplified.

According to supplement 17, a molecule-of-interest model is generated within the lattice system so as to reflect the arrangement of constituent units of the molecule of interest. By using a molecule-of-interest model that more accurately reflects the structure of the molecule of interest, the accuracy of estimating the conformation of the molecule of interest can be further improved.

According to supplement 18, a molecule-of-interest model using coarse-grained particles is generated so as to reflect the arrangement of main chains and side chains. By using a molecule-of-interest model that more accurately reflects the structure of the molecule of interest, the accuracy of estimating the conformation of the molecule of interest can be further improved.

According to supplement 19, the molecule-of-interest model is generated so as to satisfy an inttramolecular constraint regarding the positional relationship between a plurality of constituent units in the molecule-of-interest model or a cyclization constraint, so that a molecule-of-interest model consistent with the behavior of the molecule of interest in the real world is obtained. By using the molecule-of-interest model, the conformation of the molecule of interest can be more accurately estimated.

According to supplement 20, the conformation of a peptide capable of binding to a target protein (a peptide of interest) can be more accurately estimated. By accurately estimating the conformation, the binding mode of the peptide to the target protein can be estimated. This estimation can contribute to the design of a peptide that is expected to have improved drug efficacy.

According to supplement 23, since at least half of a plurality of constituent units of the molecule of interest are represented by a plurality of coarse-grained particles, a molecule-of-interest model representing the structure of the molecule of interest more precisely is generated. By using the molecule-of-interest model, the conformation of the molecule of interest can be more precisely estimated.

According to supplement 24, all of the plurality of constituent units of the molecule of interest are represented by a plurality of coarse-grained particles, so that a molecule-of-interest model representing the structure of the molecule of interest more precisely is generated. By using the molecule-of-interest model, the conformation of the molecule of interest can be more precisely estimated.

According to supplement 25, at least half of a plurality of constituent units of the molecule of interest are represented by two coarse-grained particles. By using the molecule-of-interest model, a balance can be achieved between the reduction of the time of calculation of interaction energy and precise estimation of the conformation.

According to supplement 26, all of the plurality of constituent units of the molecule of interest are represented by two coarse-grained particles. By using the molecule-of-interest model, a balance can be achieved between the reduction of the time of calculation of interaction energy and precise estimation of the conformation.

According to supplement 27, at least half of a plurality of constituent units of the molecule of interest are represented by two coarse-grained particles representing a main chain and a side chain, so that a molecule-of-interest model representing the structure or the function of the constituent units is generated. By using the molecule-of-interest model, the conformation of the molecule of interest can be estimated more precisely and more accurately.

According to supplement 28, all of the plurality of constituent units of the molecule of interest are represented by two coarse-grained particles representing a main chain and a side chain, so that a molecule-of-interest model representing the structure or the function of the constituent units is generated. By using the molecule-of-interest model, the conformation of the molecule of interest can be estimated more precisely and more accurately.

According to supplement 29, a molecule-of-interest model using coarse-grained particles is generated so as to reflect the arrangement of main chains and side chains. By using a molecule-of-interest model that more accurately reflects the structure of the molecule of interest, the accuracy of estimating the conformation of the molecule of interest can be further improved.

According to supplement 30, a molecule-of-interest model using coarse-grained particles is generated so as to reflect the arrangement of main chains and side chains. By using a molecule-of-interest model that more accurately reflects the structure of the molecule of interest, the accuracy of estimating the conformation of the molecule of interest can be further improved.

Examples

Examples of the techniques disclosed herein will be described, but the techniques disclosed herein are in no way limited to these Examples.

[Example 1]

(Object to be calculated)

Coordinate data of the crystal structure of a complex in which a cyclic peptide having a splicing inhibitory action is bound to the UHM (U2AF homology motif) domain of SPF45 (splicing factor 45) protein has been registered in Protein Data Bank (PDB) (https://www.rcsb.org/). The identifier of the crystal structure in PDB (PDB ID) is "SLSO". The crystal structure "5LSO" is composed of four polymers. A-Chain and B-chain each correspond to the UHM domain of SPF45, and C-chain and D-chain each correspond to the cyclic peptide. The cyclic peptide as C-chain is bound to the UHM domain of SPF45 protein as A-chain, and the cyclic peptide as D-chain is bound to the UHM domain of SPF45 protein as B-chain. Coordinate data of a complex model of A-chain and C-chain of the crystal structure "5LSO" was used for verification.

(Target protein)

Among amino acid residues forming a target protein, amino acid residues containing an atom positioned within 5 Å of any of atoms forming Chain C of the crystal structure "5LSO" were extracted. The extracted amino acid residues were a total of 18 amino acid residues identified as Leu309, Met312, Val313, Asp319, Asp321, Leu322, Glu325, Thr326, Glu329, Cys330, Leu372, Arg375, Tyr376, Phe377, Gly378, Gly379, Arg380 and Val382. With respect to these amino acid residues, a docking simulation of the peptides was performed.

(Coarse-graining of target protein)

Among the extracted amino acid residues, Va1313, Thr326, Cys330, Gly378, Gly379 and Val382 were approximated by one coarse-grained particle, and the remaining amino acid residues were approximated by two coarse-grained particles. Ultimately, the target protein was approximated by a total of 30 coarse-grained particles.

When the amino acid residue was approximated by one coarse-grained particle, the coarse-grained particle was arranged at the position of the center of gravity of all heavy atoms forming the amino acid residue. When the amino acid residue was approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Lys-Ser-Arg-Trp-Asp-Glu. The amine of the side chain of Lys1 and the carboxyl group on the side chain of Glu6 form an amide bond, which cyclizes the peptide.

(Coarse-graining of peptide of interest)

Among the amino acid residues forming the peptide of interest, Ser2 was approximated by one coarse-grained particle, and the remaining amino acid residues were approximated by two coarse-grained particles. Ultimately, the peptide of interest was approximated by a total of 11 coarse-grained particles.
For Ser2, the coarse-grained particle was arranged at the position of the center of gravity of all its constituent heavy atoms. For amino acid residues other than Ser2, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

In a list of orthogonal coordinates showing the positions of all coarse-grained particles forming a target protein, the maximum value and the minimum value in the x-axis direction are called max_X and min_X, respectively. Similarly, in the list, the maximum value and the minimum value in the y-axis direction are called max_Y and min_Y, respectively, and the maximum value and the minimum value in the z-axis direction are called max_Z and min_Z, respectively. Assume that orthogonal coordinates showing the position of any coarse-grained particle are represented by (X, Y, Z). The coarse-grained particle was rearranged at a position shown by the following coordinates (4).
[Equation 4]

$$\left( \frac{\sqrt{3}}{3.8}\left( X - \frac{max\_X + min\_X}{2} \right), \frac{\sqrt{3}}{3.8}\left( Y - \frac{max\_Y + min\_Y}{2} \right), \frac{\sqrt{3}}{3.8}\left( Z - \frac{max\_Z + min\_Z}{2} \right) \right) \quad \ldots (4)$$

This operation was applied to all coarse-grained particles forming the target protein and all coarse-grained particles forming the peptide of interest, thereby rearranging coarse-grained models for each of the target protein and the peptide of interest.
In a list of orthogonal coordinates showing the positions of coarse-grained particles forming a target protein after rearrangement, the minimum value in the x-axis direction, whose decimal fraction is rounded up in a negative direction, is called min_X'. Similarly, in the list, the minimum value in the y-axis direction, whose decimal fraction is rounded up in a negative direction, is called min_Y', and the minimum value in the z-axis direction, whose decimal fraction is rounded up in a negative direction, is called min_Z'. Assume that orthogonal coordinates showing the position of any coarse-grained particle after rearrangement are represented by (X', Y', Z'). The coarse-grained particle was rearranged at the position of (X'-min_X', Y'-min_Y', Z'-min_Z'). This operation was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

Orthogonal coordinates showing the position of the i-th coarse-grained particle forming the target protein and the peptide of interest are represented by $(x_i, y_i, z_i)$. Assume that orthogonal coordinates showing the position of any coarse-grained particle are represented by (X, Y, Z), and the total number of coarse-grained particles forming the target protein and coarse-grained particles forming the peptide of interest is represented by N. The coarse-grained particle was arranged at a position shown by the following coordinates (5).
[Equation 5]

$$\left(X - \frac{\sum_i^N x_i}{N}, \quad Y - \frac{\sum_i^N y_i}{N}, \quad Z - \frac{\sum_i^N z_i}{N}\right) \quad ...(5)$$

The coarse-grained particle arranged at the coordinates (5) was rotated around the z-axis by 1.571 rad, and the coarse-grained particle was translated in the x-axis direction by +2.533, in the y-axis direction by +2.533, and in the z-axis direction by +2.533. This operation was applied to all coarse-grained particles forming the target protein and all coarse-grained particles forming the peptide of interest. By this process, translation and rotational transfer of coarse-grained models were performed on each of the target protein and the peptide of interest.

Assume that orthogonal coordinates showing the position of any coarse-grained particle subjected to translation and rotational transfer are represented by (X', Y', Z'). The coarse-grained particle was rearranged at the position of the following coordinates (6). This operation was applied to all coarse-grained particles forming the target protein and all coarse-grained particles forming the peptide of interest, thereby rearranging coarse-grained models for each of the target protein and the peptide of interest.

[Equation 6]

$$\left(X' + \frac{\sum_i^N x_i}{N}, \quad Y' + \frac{\sum_i^N y_i}{N}, \quad Z' + \frac{\sum_i^N z_i}{N}\right) \quad ...(6)$$

(Generation of lattice structure)

A regular tetrahedral lattice was generated by setting 239 lattice points positioned on the following orthogonal coordinates. (5,5,4), (6,6,5), (6,4,3), (4,6,3), (4,4,5), (5,7,6), (7,5,6), (7,7,4), (5,3,2), (7,3,4), (7,5,2), (3,5,2), (3,7,4), (5,7,2), (3,3,4), (3,5,6), (5,3,6), (6,8,7), (4,8,5), (4,6,7), (5,9,8), (7,7,8), (7,9,6), (3,9,6), (5,9,4), (3,7,8), (5,5,8), (8,6,7), (8,4,5), (6,4,7), (9,5,8), (9,7,6), (9,3,6), (9,5,4), (7,3,8), (8,8,5), (8,6,3), (6,8,3), (9,9,4), (9,7,2), (7,9,2), (6,2,1), (4,4,1), (4,2,3), (5,1,0), (7,1,2), (7,3,0), (3,3,0), (5,5,0), (3,1,2), (5,1,4), (8,2,3), (6,2,5), (9,1,4), (9,3,2), (7,1,6), (8,4,1), (6,6,1), (9,5,0), (7,7,0), (2,6,1), (2,4,3), (1,5,0), (1,7,2), (3,7,0), (1,3,2), (1,5,4), (2,8,3), (2,6,5), (1,9,4), (3,9,2), (1,7,6), (4,8,1), (5,9,0), (2,2,5), (1,1,4), (1,3,6), (3,1,6), (2,4,7), (1,5,8), (3,3,8), (4,2,7), (5,1,8), (6,10,9), (4,10,7), (4,8,9), (5,11,10), (7,9,10), (7,11,8), (3,11,8), (5,11,6), (3,9,10), (5,7,10), (8,8,9), (6,6,9), (9,7,10), (9,9,8), (7,5,10), (8,10,7), (6,10,5), (9,11,6), (7,11,4), (2,10,5), (2,8,7), (1,11,6), (3,11,4), (1,9,8), (4,10,3), (5,11,2), (2,6,9), (1,7,10), (3,5,10), (4,4,9), (5,3,10), (10,6,9), (10,4,7), (8,4,9), (11,5,10), (11,7,8), (11,3,8), (11,5,6), (9,3,10), (10,8,7), (10,6,5), (11,9,6), (11,7,4), (10,2,5), (8,2,7), (11,1,6), (11,3,4), (9,1,8), (10,4,3), (11,5,2), (6,2,9), (7,1,10), (10,10,5), (10,8,3), (8,10,3), (11,11,4), (11,9,2), (9,11,2), (10,6,1), (8,8,1), (11,7,0), (9,9,0), (6,10,1), (7,11,0), (6,0,-1), (4,2,-1), (4,0,1), (5,-1,-2), (7,-1,0), (7,1,-2), (3,1,-2), (5,3,-2), (3,-1,0), (5,-1,2), (8,0,1), (6,0,3), (9,-1,2), (9,1,0), (7,-1,4), (8,2,-1), (6,4,-1), (9,3,-2), (7,5,-2), (2,4,-1), (2,2,1), (1,3,-2), (3,5,-2), (1,1,0), (4,6,-1), (5,7,-2), (2,0,3), (1,-1,2), (3,-1,4), (4,0,5), (5,-1,6), (10,0,3), (8,0,5), (11,-1,4), (11,1,2), (9,-1,6), (10,2,1), (11,3,0), (6,0,7), (7,-1,8), (10,4,-1), (8,6,-1), (11,5,-2), (9,7,-2), (6,8,-1), (7,9,-2), (0,6,-1), (0,4,1), (-1,5,-2), (-1,7,0), (1,7,-2), (-1,3,0), (-1,5,2), (0,8,1), (0,6,3), (-1,9,2), (1,9,0), (-1,7,4), (2,8,-1), (3,9,-2), (0,2,3), (-1,1,2), (-1,3,4), (0,4,5), (-1,5,6), (0,10,3), (0,8,5), (-1,11,4), (1,11,2), (-1,9,6), (2,10,1), (3,11,0), (0,6,7), (-1,7,8), (4,10,-1), (5,11,-2), (0,0,5), (-1,-1,4), (-1,1,6), (1,-1,6), (0,2,7), (-1,3,8), (1,1,8), (2,0,7), (3,-1,8), (0,4,9), (-1,5,10), (1,3,10), (2,2,9), (3,1,10), (4,0,9), (5,-1,10)

(Objective function)

The total interaction energy of the peptide of interest was calculated using the above equations (1) to (3), and equation (3) was used as an objective function for mathematical optimization. In equations (1) and (2), a value shown by the MJ matrix was used as energy of interaction between units, and the threshold Td for determining variables $a_{ij}$ and $b_{ik}$ were set as below.

[Equation 7]

$$6.5 \times \frac{\sqrt{3}}{3.8}$$

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.

(2) A coarse-grained particle corresponding to the main chain of the i-th amino acid residue of the peptide of interest and a coarse-grained particle corresponding to the main chain of the (i+1)-th amino acid residue of the peptide of interest must be placed at two lattice points adjacent to each other.

(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.

(4) For the peptide of interest, a coarse-grained particle corresponding to the side chain of Lys1 and a coarse-grained particle corresponding to the side chain of Glu6 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

[Equation 8]

$$2.5 \times \frac{\sqrt{3}}{3.8}$$

(Mathematical optimization)

For performing mathematical optimization including calculation of interaction energy, OR-Tools version 9.5.223 7(https://pypi.org/project/ortools/9.5.223 7/), one of constraint programming methods, was used. Using OR-Tools, a coarse-grained model in which the target protein was rearranged by translation and rotational transfer was input. An arrangement pattern for coarse-grained particles of the peptide of interest, which gives the minimum value of the objective variable $E_{total}$ subject to the constraint condition was ultimately obtained as a docking model. The minimum value of the objective variable $E_{total}$ was -193.44.

(Verification)

The consistency of the obtained docking model with a model obtained by coarse-graining of Chain C of the crystal structure "5LSO" was verified. As an index of the verification, a root mean square deviation (RMSD) represented by the following equation (7) was calculated.

[Equation 9]

$$\text{RMSD} = \frac{3.8}{\sqrt{3}} \times \sqrt{\frac{\sum_i^N d_i{}^2}{N}} \quad \text{... (7)}$$

Here, the variable $d_i$ represents a Euclidean distance between the position of the i-th coarse-grained particle in the docking model and the position of the i-th coarse-grained particle in a rearranged crystal structure through translation and rotational transfer. N represents the total number of coarse-grained particles forming the peptide of interest. As a result, a docking model with an RMSD of 2.87 Å with respect to the rearranged crystal structure was obtained.

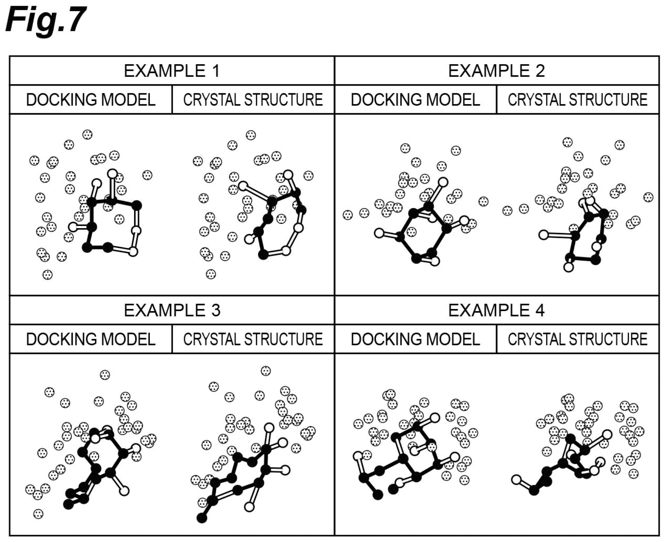

The docking model and the rearranged crystal structure in Example 1 are shown in Figure 7. The shaded circle denotes a coarse-grained particle of the target protein. The black circle denotes a coarse-grained particle corresponding to the main chain of the peptide of interest, and the white circle denotes a coarse-grained particle corresponding to the side chain of the peptide of interest.

[Example 2]

(Object to be calculated)

Coordinate data of the crystal structure of a complex between HIV-1 (human immunodeficiency virus type 1) integrase protein and a cyclic peptide has been registered in PDB. The identifier of the crystal structure in PDB is "3WNE". The crystal structure "3WNE" is composed of four polymers. A-Chain and B-chain each correspond to the HIV-1 integrase protein, and C-chain and D-chain each correspond to the cyclic peptide. The cyclic peptide as C-chain and the cyclic peptide as D-chain are both bound to the HIV-1 integrase protein dimer composed of A-chain and B-chain. Coordinate data of a complex model of A-chain, B-chain and C-chain of the crystal structure "3WNE" was used for verification.

(Target protein)

Among amino acid residues forming a target protein, amino acid residues containing an atom positioned within 5 Å of any of atoms forming C-chain of the crystal structure "3WNE" were extracted. The extracted amino acid residues were identified as Asp167, Gln168, Ala169, Glu170, His171, Thr174 and Met178 for A-chain, and Gln95, Leu102, Thr125, Ala128, Ala129 and Trp132 for B-chain, for a total of 13 amino acid residues. With respect to these amino acid residues, a docking simulation of the cyclic peptides was performed.

(Coarse-graining of target protein)

Each of the extracted amino acid residues was approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 26 coarse-grained particles.

(Peptide of interest)

The amino acid sequence of the peptide of interest was Pro-Lys-Ile-Asp-Asn-Gly. The amine of the main chain of Pro1 and the carboxyl group on the main chain of Gly6 form an amide bond, thereby cyclizing the peptide.

(Coarse-graining of peptide of interest)

Among the amino acid residues forming the peptide of interest, Gly6 was approximated by one coarse-grained particle, and the remaining amino acid residues were approximated by two coarse-grained particles. Ultimately, the peptide of interest was approximated by a total of 11 coarse-grained particles.
For Gly6, the coarse-grained particle model was arranged at the position of the center of gravity of all of its constituent heavy atoms. In amino acid residues other than Gly6, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement to the position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 3.142 rad, and rotated around the z-axis by 4.712 rad. Thereafter, the coarse-grained particle was translated in the y-axis direction by +1.267, and in the z-axis direction by +2.533. This operation was applied to all coarse-grained particles forming the target protein and all coarse-grained particles forming the peptide of interest, thereby performing translation and rotational transfer of the coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 147 lattice points positioned on the following orthogonal coordinates.
(4,5,3), (5,6,4), (5,4,2), (3,6,2), (3,4,4), (4,7,5), (6,5,5), (6,7,3), (5,8,6), (3,8,4), (3,6,6), (7,6,6), (7,4,4), (5,4,6), (7,8,4), (7,6,2), (5,8,2), (4,3,1), (6,3,3), (6,5,1), (5,2,0), (3,4,0), (3,2,2), (7,2,2), (5,2,4), (7,4,0), (5,6,0), (2,5,1), (2,7,3), (4,7,1), (1,6,0), (1,4,2), (1,8,2), (1,6,4), (3,8,0), (2,3,3), (2,5,5), (4,3,5), (1,2,4), (1,4,6), (3,2,6), (4,9,7), (6,7,7), (6,9,5), (5,10,8), (3,10,6), (3,8,8), (7,8,8), (5,6,8), (7,10,6), (5,10,4), (2,9,5), (4,9,3), (1,10,4), (1,8,6), (3,10,2), (2,7,7), (4,5,7), (1,6,8), (3,4,8), (8,5,7), (8,7,5), (9,6,8), (9,4,6), (7,4,8), (9,8,6), (9,6,4), (8,3,5), (8,5,3), (9,2,4), (7,2,6), (9,4,2), (6,3,7), (5,2,8), (8,9,3), (9,10,4), (9,8,2), (7,10,2), (8,7,1), (9,6,0), (7,8,0), (6,9,1), (5,10,0), (4,1,-1), (6,1,1), (6,3,-1), (5,0,-2), (3,2,-2), (3,0,0), (7,0,0), (5,0,2), (7,2,-2), (5,4,-2), (2,3,-1), (4,5,-1), (1,4,-2), (1,2,0), (3,6,-2), (2,1,1), (4,1,3), (1,0,2), (3,0,4), (8,1,3), (8,3,1), (9,0,2), (7,0,4), (9,2,0), (6,1,5), (5,0,6), (8,5,-1), (9,4,-2), (7,6,-2), (6,7,-1), (5,8,-2), (0,5,-1), (0,7,1), (2,7,-1), (-1,6,-2), (-1,4,0), (-1,8,0), (-1,6,2), (1,8,-2), (0,3,1), (0,5,3), (-1,2,2), (-1,4,4), (0,9,3), (2,9,1), (-1,10,2), (-1,8,4), (1,10,0), (0,7,5), (-1,6,6), (4,9,-1), (3,10,-2), (0,1,3), (0,3,5), (2,1,5), (-1,0,4), (-1,2,6), (1,0,6), (0,5,7), (2,3,7), (-1,4,8), (1,2,8), (4,1,7), (3,0,8)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Pro 1 and a coarse-grained particle corresponding to Gly6 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

[Equation 10]

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -166.61.

(Verification)

By the same technique as in Example 1, the consistency of the obtained docking model with a model obtained by coarse-graining of C-chain of the crystal structure "3WNE" was verified. As a result, a docking model with an RMSD of 3.95 Å with respect to the rearranged crystal structure was obtained. The docking model and the rearranged crystal structure in Example 2 are shown in Figure 7. The representation of each type of circle is as in Example 1.

[Example 3]

(Object to be calculated)

Coordinate data of the crystal structure of a complex between the angiotensin II peptide and the Fab (fragment antigen-binding) region of an antibody recognizing the peptide has been registered in PDB. The identifier of the crystal structure in PDB is "2CK0". The crystal structure "2CK0" is composed of three polymers. L-chain and H-chain each correspond to the Fab region of the antibody, and P-chain corresponds to the angiotensin II peptide. Coordinate data of a complex model of L-chain, H-chain and P-chain of the crystal structure "2CK0" was used for verification.

(Target protein)

Among amino acid residues forming a target protein, amino acid residues containing an atom positioned within 5 Å of any of atoms forming P-chain of the crystal structure "2CK0" were extracted. The extracted amino acid residues were identified as His31, Tyr32, Ser91, Tyr92, Asn93, Leu94 and Tyr95 for L-chain, and Asn35, Trp47, Arg50, Arg52, Gly52C, Phe53, Asn54, Ala56, Tyr58, Asp100 and Gly101 for H-chain, for a total of 18 amino acid residues. With respect to these amino acid residues, a docking simulation of the peptides was performed.

(Coarse-graining of target protein)

Among the extracted amino acid residues, Ser91 of L-chain and Gly52C, Ala56 and Gly101 of H-chain were approximated by one coarse-grained particle, and the remaining amino acid residues were approximated by two coarse-grained particles. Ultimately, the target protein was approximated by a total of 32 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Cys-Lys-Glu-Trp-Leu-Ser-Thr-Ala-Pro-Cys-Gly. The side chain of Cys1 and the side chain of Cys10 form a disulfide bond, thereby cyclizing the peptide.

(Coarse-graining of peptide of interest)

Among the amino acid residues forming the peptide of interest, Cys1, Ser6, Thr7, Ala8, Pro9, Cys10 and Gly11 were approximated by one coarse-grained particle, and the remaining amino acid residues were approximated by two coarse-grained particles. Ultimately, the peptide of interest was approximated by a total of 15 coarse-grained particles.

For Cys1, Ser6, Thr7, Ala8, Pro9, Cys10 and Gly11, the coarse-grained particle model was arranged at the position of the center of gravity of all of its constituent heavy atoms. In amino acid residues other than Cys1, Ser6, Thr7, Ala8, Pro9, Cys10 and Gly11, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 3.142 rad, and rotated around the z-axis by 3.142 rad. Thereafter, the coarse-grained particle was translated in the x-axis direction by +2.533, and in the y-axis direction by +1.267. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1,11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.

(4) For the peptide of interest, a coarse-grained particle corresponding to Cys1 and a coarse-grained particle corresponding to Cys10 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

[Equation 11]

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -307.34.

(Verification)

By the same technique as in Example 1, the consistency of the obtained docking model with a model obtained by coarse-graining of P-chain of the crystal structure "2CK0" was verified. As a result, a docking model with an RMSD of 5.45 Å with respect to the rearranged crystal structure was obtained. The docking model and the rearranged crystal structure in Example 3 are shown in Figure 7. The representation of each type of circle is as in Example 1.

[Example 4]

(Object to be calculated)

Coordinate data of the crystal structure of a complex in which the Oxytocin peptide is bound to Neurophysin protein has been registered in PDB. The identifier of the crystal structure in PDB is "1NPO". The crystal structure "1NPO" is composed of four polymers. A-Chain and C-chain each correspond to Neurophysin, and B-chain and D-chain each correspond to the Oxytocin peptide. The Oxytocin peptide as B-chain is bound to Neurophysin as A-chain, and Oxytocin as D-chain is bound to Neurophysin as C-chain. Coordinate data of a complex model of A-chain and B-chain of the crystal structure "1NPO" was used for verification.

(Target protein)

Among amino acid residues forming a target protein, amino acid residues containing an atom positioned a within 5 Å of any of atoms forming B-chain of the crystal structure "1NPO" were extracted. The extracted amino acid residues were a total of 21 amino acid residues identified as Leu5, Leu7, Arg8, Cys10, Cys21, Phe22, Gly23, Pro24, Cys44, Gln45, Glu47, Asn48, Leu50, Pro51, Ser52, Pro53, Cys54, Gln55, Gly65, Asn75 and Asp76. With respect to these amino acid residues, a docking simulation of the peptides was performed.

(Coarse-graining of target protein)

Among the extracted amino acid residues, Cys10, Cys21, Gly23, Pro24, Cys44, Pro51, Ser52, Pro53, Cys54 and Gly65 were approximated by one coarse-grained particle, and the remaining amino acid residues were approximated by two coarse-grained particles. Ultimately, the target protein was approximated by a total of 32 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Cys-Tyr-Ile-Gln-Asn-Cys-Pro-Leu-Gly. Cys1 and Cys6 form a disulfide bond, thereby cyclizing the peptide.

(Coarse-graining of peptide of interest)

Among the amino acid residues forming the peptide of interest, Cys1, Cys6, Pro7 and Gly9 were approximated by one coarse-grained particle, and the remaining amino acid residues were approximated by two coarse-grained particles. Ultimately, the peptide of interest was approximated by a total of 14 coarse-grained particles.

When the amino acid residue was approximated by one coarse-grained particle, the coarse-grained particle model was arranged at the position of the center of gravity of all of its constituent heavy atoms. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 1.047 rad, and rotated around the z-axis by 4.189 rad. Thereafter, the coarse-grained particle was translated in the x-axis direction by +1.267, in the y-axis direction by +2.533 and in the z-axis direction by +1.267. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(6,4,5), (7,5,6), (7,3,4), (5,5,4), (5,3,6), (6,6,7), (8,4,7), (8,6,5), (6,2,3), (8,2,5), (8,4,3), (4,4,3), (4,6,5), (6,6,3), (4,2,5), (4,4,7), (6,2,7), (7,7,8), (5,7,6), (5,5,8), (6,8,9), (8,6,9), (8,8,7), (4,8,7), (6,8,5), (4,6,9), (6,4,9), (9,5,8), (9,3,6), (7,3,8), (10,4,9), (10,6,7), (10,2,7), (10,4,5), (8,2,9), (9,7,6), (9,5,4), (7,7,4), (10,8,5), (10,6,3), (8,8,3), (7,1,2), (5,3,2), (5,1,4), (6,0,1), (8,0,3), (8,2,1), (4,2,1), (6,4,1), (4,0,3), (6,0,5), (9,1,4), (7,1,6), (10,0,5), (10,2,3), (8,0,7), (9,3,2), (7,5,2), (10,4,1), (8,6,1), (3,5,2), (3,3,4), (2,4,1), (2,6,3), (4,6,1), (2,2,3), (2,4,5), (3,7,4), (3,5,6), (2,8,5), (4,8,3), (2,6,7), (5,7,2), (6,8,1), (3,1,6), (2,0,5), (2,2,7), (4,0,7), (3,3,8), (2,4,9), (4,2,9), (5,1,8), (6,0,9), (7,9,10), (5,9,8), (5,7,10), (6,10,11), (8,8,11), (8,10,9), (4,10,9), (6,10,7), (4,8,11), (6,6,11), (9,7,10), (7,5,10), (10,6,11), (10,8,9), (8,4,11), (9,9,8), (7,9,6), (10,10,7), (8,10,5), (3,9,6), (3,7,8), (2,10,7), (4,10,5), (2,8,9), (5,9,4), (6,10,3), (3,5,10), (2,6,11), (4,4,11), (5,3,10), (6,2,11), (11,5,10), (11,3,8), (9,3,10), (12,4,11), (12,6,9), (12,2,9), (12,4,7), (10,2,11), (11,7,8), (11,5,6), (12,8,7), (12,6,5), (11,1,6), (9,1,8), (12,0,7), (12,2,5), (10,0,9), (11,3,4), (12,4,3), (7,1,10), (8,0,11), (11,9,6), (11,7,4), (9,9,4), (12,10,5), (12,8,3), (10,10,3), (11,5,2), (9,7,2), (12,6,1), (10,8,1), (7,9,2), (8,10,1), (7,-1,0), (5,1,0), (5,-1,2), (6,-2,-1), (8,-2,1), (8,0,-1), (4,0,-1), (6,2,-1), (4,-2,1), (6,-2,3), (9,-1,2), (7,-1,4), (10,-2,3), (10,0,1), (8,-2,5), (9,1,0), (7,3,0), (10,2,-1), (8,4,-1), (3,3,0), (3,1,2), (2,2,-1), (4,4,-1), (2,0,1), (5,5,0), (6,6,-1), (3,-1,4), (2,-2,3), (4,-2,5), (5,-1,6), (6,-2,7), (11,-1,4), (9,-1,6), (12,-2,5), (12,0,3), (10,-2,7), (11,1,2), (12,2,1), (7,-1,8), (8,-2,9), (11,3,0), (9,5,0), (12,4,-1), (10,6,-1), (7,7,0), (8,8,-1), (1,5,0), (1,3,2), (0,4,-1), (0,6,1), (2,6,-1), (0,2,1), (0,4,3), (1,7,2), (1,5,4), (0,8,3), (2,8,1), (0,6,5), (3,7,0), (4,8,-1), (1,1,4), (0,0,3), (0,2,5), (1,3,6), (0,4,7), (1,9,4), (1,7,6), (0,10,5), (2,10,3), (0,8,7), (3,9,2), (4,10,1), (1,5,8), (0,6,9), (5,9,0), (6,10,-1), (1,-1,6), (0,-2,5), (0,0,7), (2,-2,7), (1,1,8), (0,2,9), (2,0,9), (3,-1,8), (4,-2,9), (1,3,10), (0,4,11), (2,2,11), (3,1,10), (4,0,11), (5,-1,10), (6,-2,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.

(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.

(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.

(4) For the peptide of interest, a coarse-grained particle corresponding to the main chain of Cys1 and a coarse-grained particle corresponding to the main chain of Cys6 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

$$[\text{Equation 12}]$$

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -313.45.

(Verification)

By the same technique as in Example 1, the consistency of the obtained docking model with a model obtained by coarse-graining of B-chain of the crystal structure "1NPO" was verified. As a result, a docking model with an RMSD of 4.40 Å with respect to the rearranged crystal structure was obtained. The docking model and the rearranged crystal structure in Example 4 are shown in Figure 7. The representation of each type of circle is as in Example 1.

[Example 5]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AV9". The crystal structure "3AV9" is composed of four polymers. A-chain and B-chain each correspond to the HIV-1 integrase protein, and X-chain and Y-chain each correspond to the cyclic peptide. The cyclic peptide as X-chain is bound to the HIV-1 integrase protein dimer composed of A-chain and B-chain, and the cyclic peptide as Y-chain also is bound to the HIV-1 integrase protein dimer composed of A-chain and B-chain. Coordinate data of a complex model of A-chain, B-chain and Y-chain of the crystal structure "3AV9" was used for verification.

(Target protein)

Among amino acid residues forming a target protein, amino acid residues containing an atom positioned within 5 Å of any of atoms forming Y-chain of the crystal structure "3AV9" were extracted. The extracted amino acid residues were identified as Gln95, Thr124, Thr125, Ala128, Ala129, Trp131 and Trp132 for A-chain, and Asp167, Gln168, Ala169, Glu170, His171, Thr174 and Met178 for B-chain, for a total of 14 amino acid residues. With respect to these amino acid residues, a docking simulation of cyclic the peptides was performed.

(Coarse-graining of target protein)

The extracted amino acid residues were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 28 coarse-grained particles.

(Peptide of interest)

The amino acid sequence of the peptide of interest was Ser-Ala-Lys-Ile-Asp-Asn-Leu-Asp. The amine of the main chain of Ser1 and the carboxyl group on the main chain of Asp8 form an amide bond, thereby cyclizing the peptide.

(Coarse-graining of peptide of interest)

The amino acid residues forming the peptide of interest were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the peptide of interest was approximated by a total of 16 coarse-grained particles.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 4.712 rad, and rotated around the z-axis by 3.142 rad. Thereafter, the coarse-grained particle was translated in the x-axis direction by +1.267, and in the y-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.

(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1, 11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Ser1 and a coarse-grained particle corresponding to the main chain of Asp8 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

[Equation 13]

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -219.25.

(Verification)

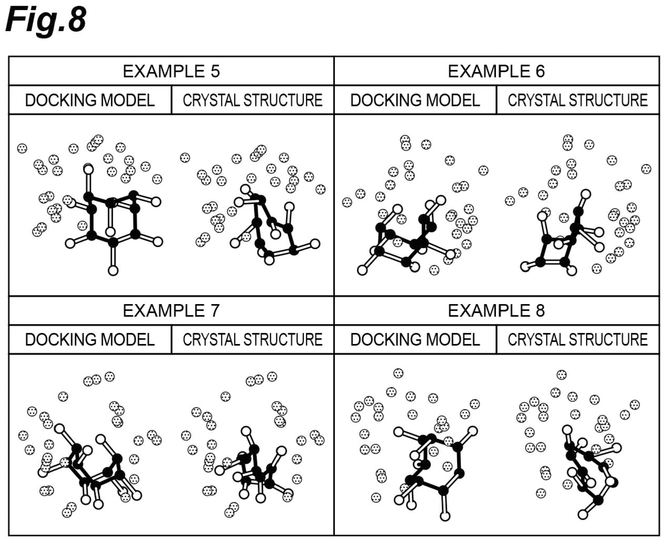

By the same technique as in Example 1, the consistency of the obtained docking model with a model obtained by coarse-graining of Y-chain of the crystal structure "3AV9" was verified. As a result, a docking model with an RMSD of 4.08 Å with respect to the rearranged crystal structure was obtained. The docking model and the rearranged crystal structure in Example 5 are shown in Figure 8. The representation of each type of circle is as in Example 1.

[Example 6]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVA". The crystal structure "3AVA" is composed of four polymers. A-chain and B-chain each correspond to the HIV-1 integrase protein, and X-chain and Y-chain each correspond to the cyclic peptide. The cyclic peptide as X-chain is bound to the HIV-1 integrase protein dimer composed of A-chain and B-chain, and the cyclic peptide as Y-chain also is bound to the HIV-1 integrase protein dimer composed of A-chain and B-chain. Coordinate data of a complex model of A-chain, B-chain and Y-chain of the crystal structure "3AVA" was used for verification.

(Target protein)

Among amino acid residues forming a target protein, amino acid residues containing an atom positioned within 5 Å of any of atoms forming Y-chain of the crystal structure "3AVA" were extracted. The extracted amino acid residues were identified as Gln95, Thr124, Thr125, Ala128, Ala129, Trp131 and Trp132 for A-chain, and Asp167, Gln168, Ala169, Glu170, His171, Thr174 and Met178 for B-chain, for a total of 14 amino acid residues. With respect to these amino acid residues, a docking simulation of cyclic the peptides was simulated.

(Coarse-graining of target protein)

The extracted amino acid residues were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 28 coarse-grained particles.

(Peptide of interest)

The amino acid sequence of the peptide of interest was Ala-Leu-Lys-Ile-Asp-Asn-Leu-Asp. The amine of the main chain of Ala1 and the carboxyl group on the main chain of Asp8 form an amide bond, thereby cyclizing the peptide.

(Coarse-graining of peptide of interest)

The amino acid residues forming the peptide of interest were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the peptide of interest was approximated by a total of 16 coarse-grained particles.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min _Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 4.712 rad, and rotated around the z-axis by 3.142 rad. Thereafter, the coarse-grained particle was translated in the x-axis direction by +2.533, in the y-axis direction by +2.533 and in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1,11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.

(4) For the peptide of interest, a coarse-grained particle corresponding to the main chain of Ala1 and a coarse-grained particle corresponding to the main chain of Asp8 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

[Equation 14]

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -298.81.

(Verification)

By the same technique as in Example 1, the consistency of the obtained docking model with a model obtained by coarse-graining of Y-chain of the crystal structure "3AVA" was verified. As a result, a docking model with an RMSD of 3.79 Å with respect to the rearranged crystal structure was obtained. The docking model and the rearranged crystal structure in Example 6 are shown in Figure 8. The representation of each type of circle is as in Example 1.

[Example 7]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVB". The crystal structure "3AVB" is composed of four polymers. A-chain and B-chain each correspond to the HIV-1 integrase protein, and X-chain and Y-chain each correspond to the cyclic peptide. The cyclic peptide as X-chain is bound to the HIV-1 integrase protein dimer composed of A-chain and B-chain, and the cyclic peptide as Y-chain also is bound to the HIV-1 integrase protein dimer composed of A-chain and B-chain. Coordinate data of a complex model of A-chain, B-chain and Y-chain of the crystal structure "3AVB" was used for verification.

(Target protein)

Among amino acid residues forming a target protein, amino acid residues containing an atom positioned within 5 Å of any of atoms forming Y-chain of the crystal structure "3AVB" were extracted. The extracted amino acid residues were identified as Gln95, Thr124, Thr125, Ala128, Ala129, Trp131 and Trp132 for A-chain, and Asp167, Gln168, Ala169, Glu170, His171, Thr174 and Met178 for B-chain, for a total of 14 amino acid residues. With respect to these amino acid residues, a docking simulation of cyclic the peptides was performed.

(Coarse-graining of target protein)

The extracted amino acid residues were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 28 coarse-grained particles.

(Peptide of interest)

The amino acid sequence of the peptide of interest was Ser-Leu-Lys-Ile-Asp-Asn-Leu-Asp. The amine of the main chain of Ser1 and the carboxyl group on the main chain of Asp8 form an amide bond, thereby cyclizing the peptide.

(Coarse-graining of peptide of interest)

The amino acid residues forming the peptide of interest were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the peptide of interest was approximated by a total of 16 coarse-grained particles.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 4.712 rad, and rotated around the z-axis by 3.142 rad. Thereafter, the coarse-grained particle was translated in the x-axis direction by +2.533, in the y-axis direction by +2.533 and in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1,11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Serl and a coarse-grained particle corresponding to the main chain of Asp8 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

[Equation 15]

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -298.28.

(Verification)

By the same technique as in Example 1, the consistency of the obtained docking model with a model obtained by coarse-graining of Y-chain of the crystal structure "3AVB" was verified. As a result, a docking model with an RMSD of 3.83 Å with respect to the rearranged crystal structure was obtained. The docking model and the rearranged crystal structure in Example 7 are shown in Figure 8. The representation of each type of circle is as in Example 1.

[Example 8]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVI". The crystal structure "3AVI" is composed of four polymers. A-chain and B-chain each correspond to the HIV-1 integrase protein, and D-chain and F-chain each correspond to the cyclic peptide. The cyclic peptide as D-chain is bound to the HIV-1 integrase protein dimer composed of A-chain and B-chain, and the cyclic peptide as F-chain also is bound to the HIV-1 integrase protein dimer composed of A-chain and B-chain. Coordinate data of a complex model of A-chain, B-chain and F-chain of the crystal structure "3AVI" was used for verification.

(Target protein)

Among amino acid residues forming a target protein, amino acid residues containing an atom positioned within 5 Å of any of atoms forming F-chain of the crystal structure "3AVI" were extracted. The extracted amino acid residues were identified as Asp167, Gln168, Ala169, Glu170, His171, Thr174 and Met178 for A-chain, and Gln95, Ala98, Leu102, Thr124, Thr125, Ala128, Ala129, Trp131 and Trp132 for B-chain, for a total of 16 amino acid residues. With respect to these amino acid residues, a docking simulation of cyclic the peptides was performed.

(Coarse-graining of target protein)

Among the extracted amino acid residues, Ala169 and Thr174 of A-chain, and Ala98, Thr124, Thr125, Ala128 and Ala129 of B-chain were approximated by one coarse-grained particle, and the remaining amino acid residues were approximated by two coarse-grained particles. Ultimately, the target protein was approximated by a total of 25 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Ser-Leu-Lys-Ile-Asp-Asn-Met-Asp. The amine of the main chain of Serl and the carboxyl group on the main chain of Asp8 form an amide bond, thereby cyclizing the peptide.

(Coarse-graining of peptide of interest)

Among the amino acid residues forming the peptide of interest, Ser1 was approximated by one coarse-grained particle, and the remaining amino acid residues were approximated by two coarse-grained particles. Ultimately, the peptide of interest was approximated by a total of 15 coarse-grained particles.

For Serl, the coarse-grained particle model was arranged at the position of the center of gravity of all of its constituent heavy atoms. In amino acid residues other than Serl, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 1.571 rad, and rotated around the z-axis by 3.142 rad. Thereafter, the coarse-grained particle was translated in the x-axis direction by +2.533, and in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.

(4,5,5), (5,6,6), (5,4,4), (3,6,4), (3,4,6), (4,7,7), (6,5,7), (6,7,5), (4,3,3), (6,3,5), (6,5,3), (2,5,3), (2,7,5), (4,7,3), (2,3,5), (2,5,7), (4,3,7), (5,8,8), (3,8,6), (3,6,8), (4,9,9), (6,7,9), (6,9,7), (2,9,7), (4,9,5), (2,7,9), (4,5,9), (7,6,8), (7,4,6), (5,4,8), (8,5,9), (8,7,7), (8,3,7), (8,5,5), (6,3,9), (7,8,6), (7,6,4), (5,8,4), (8,9,5), (8,7,3), (6,9,3), (5,2,2), (3,4,2), (3,2,4), (4,1,1), (6,1,3), (6,3,1), (2,3,1), (4,5,1), (2,1,3), (4,1,5), (7,2,4), (5,2,6), (8,1,5), (8,3,3), (6,1,7), (7,4,2), (5,6,2), (8,5,1), (6,7,1), (1,6,2), (1,4,4), (0,5,1), (0,7,3), (2,7,1), (0,3,3), (0,5,5), (1,8,4), (1,6,6), (0,9,5), (2,9,3), (0,7,7), (3,8,2), (4,9,1), (1,2,6), (0,1,5), (0,3,7), (2,1,7), (1,4,8), (0,5,9), (2,3,9), (3,2,8), (4,1,9), (5,10,10), (3,10,8), (3,8,10), (4,11,11), (6,9,11), (6,11,9), (2,11,9), (4,11,7), (2,9,11), (4,7,11), (7,8,10), (5,6,10), (8,7,11), (8,9,9), (6,5,11), (7,10,8), (5,10,6), (8,11,7), (6,11,5), (1,10,6), (1,8,8), (0,11,7), (2,11,5), (0,9,9), (3,10,4), (4,11,3), (1,6,10), (0,7,11), (2,5,11), (3,4,10), (4,3,11), (9,6,10), (9,4,8), (7,4,10), (10,5,11), (10,7,9), (10,3,9), (10,5,7), (8,3,11), (9,8,8), (9,6,6), (10,9,7), (10,7,5), (9,2,6), (7,2,8), (10,1,7), (10,3,5), (8,1,9), (9,4,4), (10,5,3), (5,2,10), (6,1,11), (9,10,6), (9,8,4), (7,10,4), (10,11,5), (10,9,3), (8,11,3), (9,6,2), (7,8,2), (10,7,1), (8,9,1), (5,10,2), (6,11,1), (5,0,0), (3,2,0), (3,0,2), (4,-1,-1), (6,-1,1), (6,1,-1), (2,1,-1), (4,3,-1), (2,-1,1), (4,-1,3), (7,0,2), (5,0,4), (8,-1,3), (8,1,1), (6,-1,5), (7,2,0), (5,4,0), (8,3,-1), (6,5,-1), (1,4,0), (1,2,2), (0,3,-1), (2,5,-1), (0,1,1), (3,6,0), (4,7,-1), (1,0,4), (0,-1,3), (2,-1,5), (3,0,6), (4,-1,7), (9,0,4), (7,0,6), (10,-1,5), (10,1,3), (8,-1,7), (9,2,2), (10,3,1), (5,0,8), (6,-1,9), (9,4,0), (7,6,0), (10,5,-1), (8,7,-1), (5,8,0), (6,9,-1), (-1,6,0), (-1,4,2), (-2,5,-1), (-2,7,1), (0,7,-1), (-2,3,1), (-2,5,3), (-1,8,2), (-1,6,4), (-2,9,3), (0,9,1), (-2,7,5), (1,8,0), (2,9,-1), (-1,2,4), (-2,1,3), (-2,3,5), (-1,4,6), (-2,5,7), (-1,10,4), (-1,8,6), (-2,11,5), (0,11,3), (-2,9,7), (1,10,2), (2,11,1), (-1,6,8), (-2,7,9), (3,10,0), (4,11,-1), (-1,0,6), (-2,-1,5), (-2,1,7), (0,-1,7), (-1,2,8), (-2,3,9), (0,1,9), (1,0,8), (2,-1,9), (-1,4,10), (-2,5,11), (0,3,11), (1,2,10), (2,1,11), (3,0,10), (4,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)-th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Ser1 and a coarse-grained particle corresponding to the main chain of Asp8 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

[Equation 16]

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -229.84.

(Verification)

By the same technique as in Example 1, the consistency of the obtained docking model with a model obtained by coarse-graining of F-chain of the crystal structure "3AVI" was verified. As a result, a docking model with an RMSD of 4.34 Å with respect to the rearranged crystal structure was obtained. The docking model and the rearranged crystal structure in Example 8 are shown in Figure 8. The representation of each type of circle is as in Example 1.

[Example 9]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVJ". The crystal structure "3AVJ" is composed of four polymers. A-chain and B-chain each correspond to the HIV-1 integrase protein, and D-chain and F-chain each correspond to the cyclic peptide. The cyclic peptide as D-chain is bound to the HIV-1 integrase protein dimer composed of A-chain and B-chain, and the cyclic peptide as F-chain also is bound to the HIV-1 integrase protein dimer composed of A-chain and B-chain. Coordinate data of a complex model of A-chain, B-chain and F-chain of the crystal structure "3AVJ" was used for verification.

(Target protein)

Among amino acid residues forming a target protein, amino acid residues containing an atom positioned within 5 Å of any of atoms forming F-chain of the crystal structure "3AVJ" were extracted. The extracted amino acid residues were identified as Asp167, Gln168, Ala169, Glu170, His171, Thr174 and Met178 for A-chain, and Gln95, Ala98, Leu102, Thr124, Thr125, Ala128, Ala129, Trp131 and Trp132 for B-chain, for a total of 16 amino acid residues. With respect to these amino acid residues, a docking simulation of cyclic the peptides was performed.

(Coarse-graining of target protein)

Among the extracted amino acid residues, Ala169 and Thr174 of A-chain, and Ala98, Thr124, Thr125, Ala128 and Ala129 of B-chain were approximated by one coarse-grained particle, and the remaining amino acid residues were approximated by two coarse-grained particles. Ultimately, the target protein was approximated by a total of 25 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Ala-Leu-Lys-Ile-Asp-Asn-Met-Asp. The amine of the main chain of Ala1 and the carboxyl group on the main chain of Asp8 form an amide bond, thereby cyclizing the peptide.

(Coarse-graining of peptide of interest)

Among the amino acid residues forming the peptide of interest, Ala1 was approximated by one coarse-grained particle, and the remaining amino acid residues were approximated by two coarse-grained particles. Ultimately, the peptide of interest was approximated by a total of 15 coarse-grained particles.

For Ala1, the coarse-grained particle model was arranged at the position of the center of gravity of all of its constituent heavy atoms. In amino acid residues other than Ala1, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 1.571 rad, and rotated around the z-axis by 3.142 rad. Thereafter, the coarse-grained particle was translated in the x-axis direction by +2.533, and in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(4,5,5), (5,6,6), (5,4,4), (3,6,4), (3,4,6), (4,7,7), (6,5,7), (6,7,5), (4,3,3), (6,3,5), (6,5,3), (2,5,3), (2,7,5), (4,7,3), (2,3,5), (2,5,7), (4,3,7), (5,8,8), (3,8,6), (3,6,8), (4,9,9), (6,7,9), (6,9,7), (2,9,7), (4,9,5), (2,7,9), (4,5,9), (7,6,8), (7,4,6), (5,4,8), (8,5,9), (8,7,7), (8,3,7), (8,5,5), (6,3,9), (7,8,6), (7,6,4), (5,8,4), (8,9,5), (8,7,3), (6,9,3), (5,2,2), (3,4,2), (3,2,4), (4,1,1), (6,1,3), (6,3,1), (2,3,1), (4,5,1), (2,1,3), (4,1,5), (7,2,4), (5,2,6), (8,1,5), (8,3,3), (6,1,7), (7,4,2), (5,6,2), (8,5,1), (6,7,1), (1,6,2), (1,4,4), (0,5,1), (0,7,3), (2,7,1), (0,3,3), (0,5,5), (1,8,4), (1,6,6), (0,9,5), (2,9,3), (0,7,7), (3,8,2), (4,9,1), (1,2,6), (0,1,5), (0,3,7), (2,1,7), (1,4,8), (0,5,9), (2,3,9), (3,2,8), (4,1,9), (5,10,10), (3,10,8), (3,8,10), (4,11,11), (6,9,11), (6,11,9), (2,11,9), (4,11,7), (2,9,11), (4,7,11), (7,8,10), (5,6,10), (8,7,11), (8,9,9), (6,5,11), (7,10,8), (5,10,6), (8,11,7), (6,11,5), (1,10,6), (1,8,8), (0,11,7), (2,11,5), (0,9,9), (3,10,4), (4,11,3), (1,6,10), (0,7,11), (2,5,11), (3,4,10), (4,3,11), (9,6,10), (9,4,8), (7,4,10), (10,5,11), (10,7,9), (10,3,9), (10,5,7), (8,3,11), (9,8,8), (9,6,6), (10,9,7), (10,7,5), (9,2,6), (7,2,8), (10,1,7), (10,3,5), (8,1,9), (9,4,4), (10,5,3), (5,2,10), (6,1,11), (9,10,6), (9,8,4), (7,10,4), (10,11,5), (10,9,3), (8,11,3), (9,6,2), (7,8,2), (10,7,1), (8,9,1), (5,10,2), (6,11,1), (5,0,0), (3,2,0), (3,0,2), (4,-1,-1), (6,-1,1), (6,1,-1), (2,1,-1), (4,3,-1), (2,-1,1), (4,-1,3), (7,0,2), (5,0,4), (8,-1,3), (8,1,1), (6,-1,5), (7,2,0), (5,4,0), (8,3,-1), (6,5,-1), (1,4,0), (1,2,2), (0,3,-1), (2,5,-1), (0,1,1), (3,6,0), (4,7,-1), (1,0,4), (0,-1,3), (2,-1,5), (3,0,6), (4,-1,7), (9,0,4), (7,0,6), (10,-1,5), (10,1,3), (8,-1,7), (9,2,2), (10,3,1), (5,0,8), (6,-1,9), (9,4,0), (7,6,0), (10,5,-1), (8,7,-1), (5,8,0), (6,9,-1), (-1,6,0), (-1,4,2), (-2,5,-1), (-2,7,1), (0,7,-1), (-2,3,1), (-2,5,3), (-1,8,2), (-1,6,4), (-2,9,3), (0,9,1), (-2,7,5), (1,8,0), (2,9,-1), (-1,2,4), (-2,1,3), (-2,3,5), (-1,4,6), (-2,5,7), (-1,10,4), (-1,8,6), (-2,11,5), (0,11,3), (-2,9,7), (1,10,2), (2,11,1), (-1,6,8), (-2,7,9), (3,10,0), (4,11,-1), (-1,0,6), (-2,-1,5), (-2,1,7), (0,-1,7), (-1,2,8), (-2,3,9), (0,1,9), (1,0,8), (2,-1,9), (-1,4,10), (-2,5,11), (0,3,11), (1,2,10), (2,1,11), (3,0,10), (4,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.

(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.

(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.

(4) For the peptide of interest, a coarse-grained particle corresponding to the main chain of Ala1 and a coarse-grained particle corresponding to the main chain of Asp8 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

[Equation 17]

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -235.20.

(Verification)

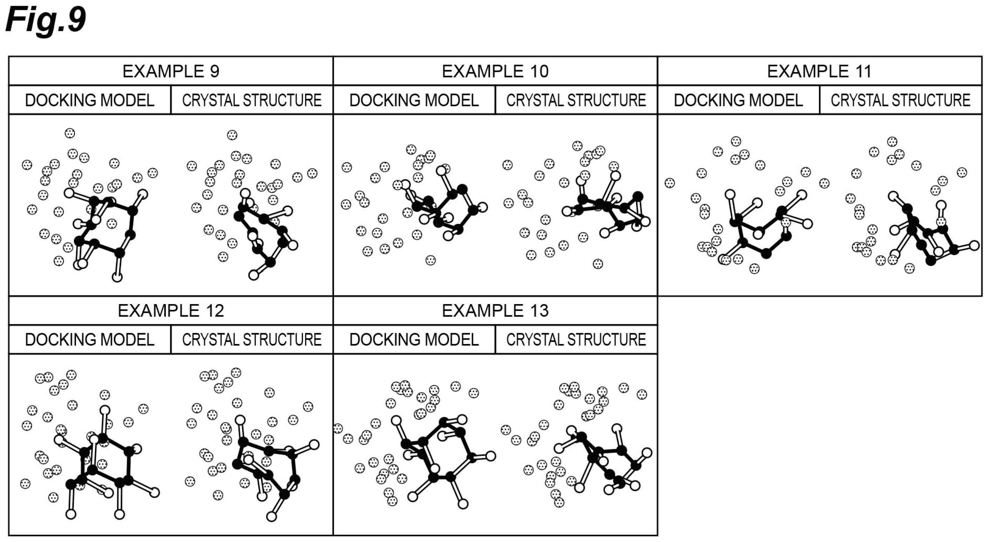

By the same technique as in Example 1, the consistency of the obtained docking model with a model obtained by coarse-graining of F-chain of the crystal structure "3AVJ" was verified. As a result, a docking model with an RMSD of 4.33 Å with respect to the rearranged crystal structure was obtained. The docking model and the rearranged crystal structure in Example 9 are shown in Figure 9. The representation of each type of circle is as in Example 1.

[Example 10]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVK". The crystal structure "3AVK" is composed of four polymers. A-chain and B-chain each correspond to the HIV-1 integrase protein, and D-chain and F-chain each correspond to the cyclic peptide. The cyclic peptide as D-chain is bound to the HIV-1 integrase protein dimer composed of A-chain and B-chain, and the cyclic peptide as F-chain also is bound to the HIV-1 integrase protein dimer composed of A-chain and B-chain. Coordinate data of a complex model of A-chain, B-chain and F-chain of the crystal structure "3AVK" was used for verification.

(Target protein)

Among amino acid residues forming a target protein, amino acid residues containing an atom positioned within 5 Å of any of atoms forming F-chain of the crystal structure "3AVK" were extracted. The extracted amino acid residues were identified as Asp167, Gln168, Ala169, Glu170, His171, Thr174 and Met178 for A-chain, and Gln95, Ala98, Leu102, Thr124, Thr125, Ala128, Ala129, Trp131 and Trp132 for B-chain, for a total of 16 amino acid residues. With respect to these amino acid residues, a docking simulation of cyclic the peptides was performed.

(Coarse-graining of target protein)

Among the extracted amino acid residues, Ala169 and Thr174 of A-chain, and Ala98, Thr124, Thr125, Ala128 and Ala129 of B-chain were approximated by one coarse-grained particle, and the remaining amino acid residues were approximated by two coarse-grained particles. Ultimately, the target protein was approximated by a total of 25 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Ser-Leu-Lys-Ile-Asp-Asn-Glu-Asp. The amine of the main chain of Ser1 and the carboxyl group on the main chain of Asp8 form an amide bond, thereby cyclizing the peptide.

(Coarse-graining of peptide of interest)

Among the amino acid residues forming the peptide of interest, Ala1 was approximated by one coarse-grained particle, and the remaining amino acid residues were approximated by two coarse-grained particles. Ultimately, the peptide of interest was approximated by a total of 15 coarse-grained particles.

For Ser1, the coarse-grained particle model was arranged at the position of the center of gravity of all of its constituent heavy atoms. In amino acid residues other than Ser1, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 3.142 rad, and rotated around the z-axis by 1.571 rad. Thereafter, the coarse-grained particle was translated in the x-axis direction by +1.267, and in the z-axis direction by +1.267. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(4,4,5), (5,5,6), (5,3,4), (3,5,4), (3,3,6), (4,6,7), (6,4,7), (6,6,5), (4,2,3), (6,2,5), (6,4,3), (2,4,3), (2,6,5), (4,6,3), (2,2,5), (2,4,7), (4,2,7), (5,7,8), (3,7,6), (3,5,8), (4,8,9), (6,6,9), (6,8,7), (2,8,7), (4,8,5), (2,6,9), (4,4,9), (7,5,8), (7,3,6), (5,3,8), (8,4,9), (8,6,7), (8,2,7), (8,4,5), (6,2,9), (7,7,6), (7,5,4), (5,7,4), (8,8,5), (8,6,3), (6,8,3), (5,1,2), (3,3,2), (3,1,4), (4,0,1), (6,0,3), (6,2,1), (2,2,1), (4,4,1), (2,0,3), (4,0,5), (7,1,4), (5,1,6), (8,0,5), (8,2,3), (6,0,7), (7,3,2), (5,5,2), (8,4,1), (6,6,1), (1,5,2), (1,3,4), (0,4,1), (0,6,3), (2,6,1), (0,2,3), (0,4,5), (1,7,4), (1,5,6), (0,8,5), (2,8,3), (0,6,7), (3,7,2), (4,8,1), (1,1,6), (0,0,5), (0,2,7), (2,0,7), (1,3,8), (0,4,9), (2,2,9), (3,1,8), (4,0,9), (5,9,10), (3,9,8), (3,7,10), (4,10,11), (6,8,11), (6,10,9), (2,10,9), (4,10,7), (2,8,11), (4,6,11), (7,7,10), (5,5,10), (8,6,11), (8,8,9), (6,4,11), (7,9,8), (5,9,6), (8,10,7), (6,10,5), (1,9,6), (1,7,8), (0,10,7), (2,10,5), (0,8,9), (3,9,4), (4,10,3), (1,5,10), (0,6,11), (2,4,11), (3,3,10), (4,2,11), (9,5,10), (9,3,8), (7,3,10), (10,4,11), (10,6,9), (10,2,9), (10,4,7), (8,2,11), (9,7,8), (9,5,6), (10,8,7), (10,6,5), (9,1,6), (7,1,8), (10,0,7), (10,2,5), (8,0,9), (9,3,4), (10,4,3), (5,1,10), (6,0,11), (9,9,6), (9,7,4), (7,9,4), (10,10,5), (10,8,3), (8,10,3), (9,5,2), (7,7,2), (10,6,1), (8,8,1), (5,9,2), (6,10,1), (5,-1,0), (3,1,0), (3,-1,2), (4,-2,-1), (6,-2,1), (6,0,-1), (2,0,-1), (4,2,-1), (2,-2,1), (4,-2,3), (7,-1,2), (5,-1,4), (8,-2,3), (8,0,1), (6,-2,5), (7,1,0), (5,3,0), (8,2,-1), (6,4,-1), (1,3,0), (1,1,2), (0,2,-1), (2,4,-1), (0,0,1), (3,5,0), (4,6,-1), (1,-1,4), (0,-2,3), (2,-2,5), (3,-1,6), (4,-2,7), (9,-1,4), (7,-1,6), (10,-2,5), (10,0,3), (8,-2,7), (9,1,2), (10,2,1), (5,-1,8), (6,-2,9), (9,3,0), (7,5,0), (10,4,-1), (8,6,-1), (5,7,0), (6,8,-1), (-1,5,0), (-1,3,2), (-2,4,-1), (-2,6,1), (0,6,-1), (-2,2,1), (-2,4,3), (-1,7,2), (-1,5,4), (-2,8,3), (0,8,1), (-2,6,5), (1,7,0), (2,8,-1), (-1,1,4), (-2,0,3), (-2,2,5), (-1,3,6), (-2,4,7), (-1,9,4), (-1,7,6), (-2,10,5), (0,10,3), (-2,8,7), (1,9,2), (2,10,1), (-1,5,8), (-2,6,9), (3,9,0), (4,10,-1), (-1,-1,6), (-2,-2,5), (-2,0,7), (0,-2,7), (-1,1,8), (-2,2,9), (0,0,9), (1,-1,8), (2,-2,9), (-1,3,10), (-2,4,11), (0,2,11), (1,1,10), (2,0,11), (3,-1,10), (4,-2,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Serl and a coarse-grained particle corresponding to the main chain of Asp8 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

$$[Equation\ 18]$$

$$2.5 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -194.35.

(Verification)

By the same technique as in Example 1, the consistency of the obtained docking model with a model obtained by coarse-graining of F-chain of the crystal structure "3AVK" was verified. As a result, a docking model with an RMSD of 3.92 Å with respect to the rearranged crystal structure was obtained. The docking model and the rearranged crystal structure in Example 10 are shown in Figure 9. The representation of each type of circle is as in Example 1.

[Example 11]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVL". The crystal structure "3AVL" is composed of four polymers. A-chain and B-chain each correspond to the HIV-1 integrase protein, and E-chain and F-chain each correspond to the cyclic peptide. The cyclic peptide as E-chain is bound to the HIV-1 integrase protein dimer composed of A-chain and B-chain, and the cyclic peptide as F-chain also is bound to the HIV-1 integrase protein dimer composed of A-chain and B-chain. Coordinate data of a complex model of A-chain, B-chain and F-chain of the crystal structure "3AVL" was used for verification.

(Target protein)

Among amino acid residues forming a target protein, amino acid residues containing an atom positioned within 5 Å of any of atoms forming F-chain of the crystal structure "3AVL" were extracted. The extracted amino acid residues were identified as Asp167, Gln168, Ala169, Glu170, His171, Thr174 and Met178 for A-chain, and Gln95, Thr124, Thr125, Ala128, Ala129, Trp131 and Trp132 for B-chain, for a total of 14 amino acid residues. With respect to these amino acid residues, a docking simulation of the cyclic peptides was performed.

(Coarse-graining of target protein)

Among the extracted amino acid residues, Ala169 and Thr174 of A-chain, and Thr124, Thr125, Ala128 and Ala129 of B-chain were approximated by one coarse-grained particle, and the remaining amino acid residues were approximated by two coarse-grained particles. Ultimately, the target protein was approximated by a total of 22 coarse-grained particles.

When the amino acid residue was approximated by one coarse-grained particle, the coarse-grained particle was arranged at the position of the center of gravity of all heavy atoms forming the amino acid residue. When the amino acid residue is approximated by two coarse-grained particles, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Peptide of interest)

The amino acid sequence of the peptide of interest was Ala-Thr-Lys-Ile-Asp-Asn-Leu-Asp. The amine of the main chain of Ala1 and the carboxyl group on the main chain of Asp8 form an amide bond, thereby cyclizing the peptide.

(Coarse-graining of peptide of interest)

Among the amino acid residues forming the peptide of interest, Ala1 and Thr2 were approximated by one coarse-grained particle, and the remaining amino acid residues were approximated by two coarse-grained particles. Ultimately, the peptide of interest was approximated by a total of 14 coarse-grained particles.

For Ala1 and Thr2, the coarse-grained particle model was arranged at the position of the center of gravity of all of its constituent heavy atoms. In amino acid residues other than Ala1 and Thr2, one coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 4.712 rad, and rotated around the z-axis by 1.571 rad. Thereafter, the coarse-grained particle was translated in the y-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(4,5,5), (5,6,6), (5,4,4), (3,6,4), (3,4,6), (4,7,7), (6,5,7), (6,7,5), (4,3,3), (6,3,5), (6,5,3), (2,5,3), (2,7,5), (4,7,3), (2,3,5), (2,5,7), (4,3,7), (5,8,8), (3,8,6), (3,6,8), (4,9,9), (6,7,9), (6,9,7), (2,9,7), (4,9,5), (2,7,9), (4,5,9), (7,6,8), (7,4,6), (5,4,8), (8,5,9), (8,7,7), (8,3,7), (8,5,5), (6,3,9), (7,8,6), (7,6,4), (5,8,4), (8,9,5), (8,7,3), (6,9,3), (5,2,2), (3,4,2), (3,2,4), (4,1,1), (6,1,3), (6,3,1), (2,3,1), (4,5,1), (2,1,3), (4,1,5), (7,2,4), (5,2,6), (8,1,5), (8,3,3), (6,1,7), (7,4,2), (5,6,2), (8,5,1), (6,7,1), (1,6,2), (1,4,4), (0,5,1), (0,7,3), (2,7,1), (0,3,3), (0,5,5), (1,8,4), (1,6,6), (0,9,5), (2,9,3), (0,7,7), (3,8,2), (4,9,1), (1,2,6), (0,1,5), (0,3,7), (2,1,7), (1,4,8), (0,5,9), (2,3,9), (3,2,8), (4,1,9), (5,10,10), (3,10,8), (3,8,10), (4,11,11), (6,9,11), (6,11,9), (2,11,9), (4,11,7), (2,9,11), (4,7,11), (7,8,10), (5,6,10), (8,7,11), (8,9,9), (6,5,11), (7,10,8), (5,10,6), (8,11,7), (6,11,5), (1,10,6), (1,8,8), (0,11,7), (2,11,5), (0,9,9), (3,10,4), (4,11,3), (1,6,10), (0,7,11), (2,5,11), (3,4,10), (4,3,11), (9,6,10), (9,4,8), (7,4,10), (10,5,11), (10,7,9), (10,3,9), (10,5,7), (8,3,11), (9,8,8), (9,6,6), (10,9,7), (10,7,5), (9,2,6), (7,2,8), (10,1,7), (10,3,5), (8,1,9), (9,4,4), (10,5,3), (5,2,10), (6,1,11), (9,10,6), (9,8,4), (7,10,4), (10,11,5), (10,9,3), (8,11,3), (9,6,2), (7,8,2), (10,7,1), (8,9,1), (5,10,2), (6,11,1), (5,0,0), (3,2,0), (3,0,2), (4,-1,-1), (6,-1,1), (6,1,-1), (2,1,-1), (4,3,-1), (2,-1,1), (4,-1,3), (7,0,2), (5,0,4), (8,-1,3), (8,1,1), (6,-1,5), (7,2,0), (5,4,0), (8,3,-1), (6,5,-1), (1,4,0), (1,2,2), (0,3,-1), (2,5,-1), (0,1,1), (3,6,0), (4,7,-1), (1,0,4), (0,-1,3), (2,-1,5), (3,0,6), (4,-1,7), (9,0,4), (7,0,6), (10,-1,5), (10,1,3), (8,-1,7), (9,2,2), (10,3,1), (5,0,8), (6,-1,9), (9,4,0), (7,6,0), (10,5,-1), (8,7,-1), (5,8,0), (6,9,-1), (-1,6,0), (-1,4,2), (-2,5,-1), (-2,7,1), (0,7,-1), (-2,3,1), (-2,5,3), (-1,8,2), (-1,6,4), (-2,9,3), (0,9,1), (-2,7,5), (1,8,0), (2,9,-1), (-1,2,4), (-2,1,3), (-2,3,5), (-1,4,6), (-2,5,7), (-1,10,4), (-1,8,6), (-2,11,5), (0,11,3), (-2,9,7), (1,10,2), (2,11,1), (-1,6,8), (-2,7,9), (3,10,0), (4,11,-1), (-1,0,6), (-2,-1,5), (-2,1,7), (0,-1,7), (-1,2,8), (-2,3,9), (0,1,9), (1,0,8), (2,-1,9), (-1,4,10), (-2,5,11), (0,3,11), (1,2,10), (2,1,11), (3,0,10), (4,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Ala1 and a coarse-grained particle corresponding to the main chain of Asp8 must be placed at two lattice points adjacent to each other.

(5) A coarse-grained particle of the peptide of interest must not be placed at a lattice point positioned such that the Euclidean distance between the lattice point and a coarse-grained particle forming the target protein is within the following value.

[Equation 19]

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -193.68.

(Verification)

By the same technique as in Example 1, the consistency of the obtained docking model with a model obtained by coarse-graining of F-chain of the crystal structure "3AVL" was verified. As a result, a docking model with an RMSD of 4.76 Å with respect to the rearranged crystal structure was obtained. The docking model and the rearranged crystal structure in Example 11 are shown in Figure 9. The representation of each type of circle is as in Example 1.

[Example 12]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVM". The crystal structure "3AVM" is composed of four polymers. A-chain and B-chain each correspond to the HIV-1 integrase protein, and D-chain and F-chain each correspond to the cyclic peptide. The cyclic peptide as D-chain is bound to the HIV-1 integrase protein dimer composed of A-chain and B-chain, and the cyclic peptide as F-chain also is bound to the HIV-1 integrase protein dimer composed of A-chain and B-chain. Coordinate data of a complex model of A-chain, B-chain and F-chain of the crystal structure "3AVM" was used for verification.

(Target protein)

Among amino acid residues forming a target protein, amino acid residues containing an atom positioned within 5 Å of any of atoms forming F-chain of the crystal structure "3AVM" were extracted. The extracted amino acid residues were identified as Asp167, Gln168, Ala169, Glu170, His171, Thr174 and Met178 for A-chain, and Gln95, Thr124, Thr125, Ala128, Ala129, Trp131 and Trp132 for B-chain, for a total of 14 amino acid residues. With respect to these amino acid residues, a docking simulation of the cyclic peptide was performed.

(Coarse-graining of target protein)

The extracted amino acid residues were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 28 coarse-grained particles.

(Peptide of interest)

The amino acid sequence of the peptide of interest was Ser-Arg-Lys-Ile-Asp-Asn-Leu-Asp. The amine of the main chain of Serl and the carboxyl group on the main chain of Asp8 form an amide bond, thereby cyclizing the peptide.

(Coarse-graining of peptide of interest)

The amino acid residues forming the peptide of interest were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the peptide of interest was approximated by a total of 16 coarse-grained particles.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 3.142 rad, and rotated around the z-axis by 3.142 rad. Thereafter, the coarse-grained particle was translated in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1,11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two adjacent lattice points.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two adjacent lattice points.
(4) For the peptide of interest, a coarse-grained particle corresponding to the main chain of Serl and a coarse-grained particle corresponding to the main chain of Asp8 must be placed at two adjacent lattice points.
(5) A coarse-grained particle of the peptide of interest must not be placed at a lattice point positioned such that the Euclidean distance between the lattice point and a coarse-grained particle forming the target protein is within the following value.

[Equation 20]

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -226.76.

(Verification)

By the same technique as in Example 1, the consistency of the obtained docking model with a model obtained by coarse-graining of F-chain of the crystal structure "3AVM" was verified. As a result, a docking model with an RMSD of 4.17 Å with respect to the rearranged crystal structure was obtained. The docking model and the rearranged crystal structure in Example 12 are shown in Figure 9. The representation of each type of circle is as in Example 1.

[Example 13]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3AVN". The crystal structure "3AVN" is composed of four polymers. A-chain and B-chain each correspond to the HIV-1 integrase protein, and G-chain and H-chain each correspond to the cyclic peptide. The cyclic peptide as G-chain is bound to the HIV-1 integrase protein dimer composed of A-chain and B-chain, and the cyclic peptide as H-chain also is bound to the HIV-1 integrase protein dimer composed of A-chain and B-chain. Coordinate data of a complex model of A-chain, B-chain and H-chain of the crystal structure "3AVN" was used for verification.

(Target protein)

Among amino acid residues forming a target protein, amino acid residues containing an atom positioned within 5 Å of any of atoms forming H-chain of the crystal structure "3AVN" were extracted. The extracted amino acid residues were identified as Asp167, Gln168, Ala169, Glu170, His171, Thr174 and Met178 for A-chain, and Gln95, Thr124, Thr125, Ala128, Ala129, Trp131 and Trp132 for B-chain, for a total of 14 amino acid residues. With respect to these amino acid residues, a docking simulation of the cyclic peptide was performed.

(Coarse-graining of target protein)

The extracted amino acid residues were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the target protein was approximated by a total of 28 coarse-grained particles.

(Peptide of interest)

The amino acid sequence of the peptide of interest was Ser-His-Lys-Ile-Asp-Asn-Leu-Asp. The amine of the main chain of Ser1 and the carboxyl group on the main chain of Asp8 form an amide bond, thereby cyclizing the peptide.

(Coarse-graining of peptide of interest)

The amino acid residues forming the peptide of interest were approximated by two coarse-grained particles. One coarse-grained particle corresponds to a main chain, and the other coarse-grained particle corresponds to a side chain. The coarse-grained particle corresponding to a main chain was arranged at the position of the center of gravity of heavy atoms to which the atom name of CA, C, N or O is assigned, among heavy atoms forming the amino acid residue. The coarse-grained particle corresponding to a side chain was arranged at the position of the center of gravity of heavy atoms to which an atom name other than CA, C, N and O is assigned, among heavy atoms forming the amino acid residue. Ultimately, the peptide of interest was approximated by a total of 16 coarse-grained particles.

(Scaling and rearrangement of coarse-grained models)

Scaling and rearrangement of coarse-grained models were performed as in Example 1. That is, rearrangement at a position shown by the coordinates (4) was applied to all coarse-grained particles forming the target protein and all coarse-grained particles forming the peptide of interest, thereby rearranging coarse-grained models for each of the target protein and the peptide of interest. The operation of further arranging the coarse-grained particles after rearrangement at the position of (X'-min_X',Y'-min_Y',Z'-min_Z') was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

As in Example 1, the i-th coarse-grained particle of the target protein was arranged at a position shown by the coordinates (5). The coarse-grained particle arranged at the coordinates (5) was rotated around the x-axis by 3.142 rad, and rotated around the z-axis by 3.142 rad. Thereafter, the coarse-grained particle was translated in the z-axis direction by +2.533. This operation was applied to all coarse-grained particles forming the target protein and all coarse-grained particles forming the peptide of interest, thereby performing translation and rotational transfer of coarse-grained models on each of the target protein and the peptide of interest. Subsequently, as in Example 1, rearrangement at the coordinates (6) was performed on each of all coarse-grained particles forming the target protein and each of all coarse-grained particles forming the peptide of interest, thereby rearranging coarse-grained models of each of the target protein and the peptide of interest.

(Generation of lattice structure)

A regular tetrahedral lattice was generated by setting 239 lattice points positioned on the following orthogonal coordinates.
(5,5,5), (6,6,6), (6,4,4), (4,6,4), (4,4,6), (5,7,7), (7,5,7), (7,7,5), (5,3,3), (7,3,5), (7,5,3), (3,5,3), (3,7,5), (5,7,3), (3,3,5), (3,5,7), (5,3,7), (6,8,8), (4,8,6), (4,6,8), (5,9,9), (7,7,9), (7,9,7), (3,9,7), (5,9,5), (3,7,9), (5,5,9), (8,6,8), (8,4,6), (6,4,8), (9,5,9), (9,7,7), (9,3,7), (9,5,5), (7,3,9), (8,8,6), (8,6,4), (6,8,4), (9,9,5), (9,7,3), (7,9,3), (6,2,2), (4,4,2), (4,2,4), (5,1,1), (7,1,3), (7,3,1), (3,3,1), (5,5,1), (3,1,3), (5,1,5), (8,2,4), (6,2,6), (9,1,5), (9,3,3), (7,1,7), (8,4,2), (6,6,2), (9,5,1), (7,7,1), (2,6,2), (2,4,4), (1,5,1), (1,7,3), (3,7,1), (1,3,3), (1,5,5), (2,8,4), (2,6,6), (1,9,5), (3,9,3), (1,7,7), (4,8,2), (5,9,1), (2,2,6), (1,1,5), (1,3,7), (3,1,7), (2,4,8), (1,5,9), (3,3,9), (4,2,8), (5,1,9), (6,10,10), (4,10,8), (4,8,10), (5,11,11), (7,9,11), (7,11,9), (3,11,9), (5,11,7), (3,9,11), (5,7,11), (8,8,10), (6,6,10), (9,7,11), (9,9,9), (7,5,11), (8,10,8), (6,10,6), (9,11,7), (7,11,5), (2,10,6), (2,8,8), (1,11,7), (3,11,5), (1,9,9), (4,10,4), (5,11,3), (2,6,10), (1,7,11), (3,5,11), (4,4,10), (5,3,11), (10,6,10), (10,4,8), (8,4,10), (11,5,11), (11,7,9), (11,3,9), (11,5,7), (9,3,11), (10,8,8), (10,6,6), (11,9,7), (11,7,5), (10,2,6), (8,2,8), (11,1,7), (11,3,5), (9,1,9), (10,4,4), (11,5,3), (6,2,10), (7,1,11), (10,10,6), (10,8,4), (8,10,4), (11,11,5), (11,9,3), (9,11,3), (10,6,2), (8,8,2), (11,7,1), (9,9,1), (6,10,2), (7,11,1), (6,0,0), (4,2,0), (4,0,2), (5,-1,-1), (7,-1,1), (7,1,-1), (3,1,-1), (5,3,-1), (3,-1,1), (5,-1,3), (8,0,2), (6,0,4), (9,-1,3), (9,1,1), (7,-1,5), (8,2,0), (6,4,0), (9,3,-1), (7,5,-1), (2,4,0), (2,2,2), (1,3,-1), (3,5,-1), (1,1,1), (4,6,0), (5,7,-1), (2,0,4), (1,-1,3), (3,-1,5), (4,0,6), (5,-1,7), (10,0,4), (8,0,6), (11,-1,5), (11,1,3), (9,-1,7), (10,2,2), (11,3,1), (6,0,8), (7,-1,9), (10,4,0), (8,6,0), (11,5,-1), (9,7,-1), (6,8,0), (7,9,-1), (0,6,0), (0,4,2), (-1,5,-1), (-1,7,1), (1,7,-1), (-1,3,1), (-1,5,3), (0,8,2), (0,6,4), (-1,9,3), (1,9,1), (-1,7,5), (2,8,0), (3,9,-1), (0,2,4), (-1,1,3), (-1,3,5), (0,4,6), (-1,5,7), (0,10,4), (0,8,6), (-1,11,5), (1,11,3), (-1,9,7), (2,10,2), (3,11,1), (0,6,8), (-1,7,9), (4,10,0), (5,11,-1), (0,0,6), (-1,-1,5), (-1,1,7), (1,-1,7), (0,2,8), (-1,3,9), (1,1,9), (2,0,8), (3,-1,9), (0,4,10), (-1,5,11), (1,3,11), (2,2,10), (3,1,11), (4,0,10), (5,-1,11)

(Objective function)

As in Example 1, the total interaction energy of the peptide of interest was calculated using the above equations (1) to (3), and equation (3) was used as an objective function for mathematical optimization. The utilization of the MJ matrix and the setting of the threshold Td were as in Example 1.

(Constraint condition)

A constraint condition including the following five constraints was set for generating a conformation model of the peptide of interest.

(1) All coarse-grained particles forming the peptide of interest must be placed at lattice points of a regular tetrahedral lattice, and a plurality of coarse-grained particles must not be placed at one lattice point.
(2) A coarse-grained particle corresponding to the main chain of the i-th amino acid residue of the peptide of interest and a coarse-grained particle corresponding to the main chain of the (i+1)th amino acid residue of the peptide of interest must be placed at two lattice points adjacent to each other.
(3) When the i-th amino acid residue of the peptide of interest is approximated by two coarse-grained particles, a coarse-grained particle corresponding to the main chain of the amino acid residue and a coarse-grained particle corresponding to the side chain of the amino acid residue must be placed at two lattice points adjacent to each other.
(4) For the peptide of interest, a coarse-grained particle corresponding to the main chain of Ser1 and a coarse-grained particle corresponding to the main chain of Asp8 must be placed at two lattice points adjacent to each other.
(5) A coarse-grained particle of the peptide of interest must not be placed at a lattice point positioned such that the Euclidean distance between the lattice point and a coarse-grained particle forming the target protein is within the following value.

$$[Equation\ 21]$$

$$3 \times \frac{\sqrt{3}}{3.8}$$

(Mathematical optimization)

By the same process as in Example 1, an arrangement pattern for coarse-grained particles of the peptide of interest, which gives the minimum value of the objective variable $E_{total}$ subject to the constraint condition was obtained as a docking model. The minimum value of the objective variable $E_{total}$ was -226.35.

(Verification)

By the same technique as in Example 1, the consistency of the obtained docking model with a model obtained by coarse-graining of H-chain of the crystal structure "3AVN" was verified. As a result, a docking model with an RMSD of 4.04 Å with respect to the rearranged crystal structure was obtained. The docking model and the rearranged crystal structure in Example 13 are shown in Figure 9. The representation of each type of circle is as in Example 1.

[Comparative Example 1]

(Object to be calculated)

Coordinate data of the crystal structure of a complex of HIV-1 (human immunodeficiency virus type 1) integrase protein and a cyclic peptide has been registered in PDB. The identifier of the crystal structure in PDB is "3WNE". The crystal structure "3WNE" is composed of four polymers. A-Chain and B-chain each correspond to the HIV-1 integrase protein, and C-chain and D-chain each correspond to the cyclic peptide. Both the cyclic peptide of C-chain and the cyclic peptide of D-chain are bound to the HIV-1 integrase protein dimer composed of A-chain and B-chain. Coordinate data of a complex model of A-chain, B-chain and C-chain of the crystal structure "3WNE" was used for verification.

(Target protein)

Among amino acid residues forming a target protein, amino acid residues containing an atom positioned within 5 Å of any of atoms forming C-chain of the crystal structure "3WNE" were extracted. The extracted amino acid residues were identified as Asp167, Gln168, Ala169, Glu170, His171, Thr174 and Met178 for A-chain, and Gln95, Leu102, Thr125, Ala128, Ala129 and Trp132 for B-chain, for a total of 13 amino acid residues. With respect to these amino acid residues, a docking simulation of the cyclic peptide was performed.

(Coarse-graining of target protein)

Each of the extracted amino acid residues was approximated by one coarse-grained particle. For each amino acid residue, a coarse-grained particle model was arranged at the position of the center of gravity of all heavy atoms forming the amino acid residue, whereby the target protein was ultimately approximated by 13 coarse-grained particles.

(Peptide of interest)

The amino acid sequence of the peptide of interest was Pro-Lys-Ile-Asp-Asn-Gly. The amine of the main chain of Pro1 and the carboxyl group on the main chain of Gly6 form an amide bond, thereby cyclizing the peptide.

(Coarse-graining of peptide of interest)

Each of the amino acid residues forming the peptide of interest was approximated by one coarse-grained particle. Ultimately, the peptide of interest was approximated by a total of 6 coarse-grained particles. The coarse-grained particle model was arranged at the position of the center of gravity of all heavy atoms constituting the peptide of interest.

(Scaling and rearrangement of coarse-grained models)

In a list of orthogonal coordinates showing the positions of all coarse-grained particles forming a target protein, the maximum of the x-axis value and the minimum of the x-axis value are called max_X and min_X, respectively, the maximum of the y-axis value and the minimum of the y-axis value are called max_Y and min_Y, respectively, and the maximum of the z-axis value and the minimum of the z-axis value are called max_Z and min_Z, respectively. Assume that orthogonal coordinates showing the position of any coarse-grained particle are (X, Y, Z). The coarse-grained particle was rearranged at a position shown by the following coordinates (8).
[Equation 22]

$$\left(\frac{\sqrt{3}}{3.8}\left(X - \frac{max\_X + min\_X}{2}\right), \frac{\sqrt{3}}{3.8}\left(Y - \frac{max\_Y + min\_Y}{2}\right), \frac{\sqrt{3}}{3.8}\left(Z - \frac{max\_Z + min\_Z}{2}\right)\right) \quad ... (8)$$

This operation was applied to all coarse-grained particles forming the target protein and all coarse-grained particles forming the peptide of interest, thereby rearranging coarse-grained models for each of the target protein and the peptide of interest.
In a list of orthogonal coordinates showing the positions of coarse-grained particles forming a target protein after rearrangement, the minimum value in the x-axis direction, whose decimal fraction is rounded up in a negative direction, is called min_X'. Similarly, in the list, the minimum value in the y-axis direction, whose decimal fraction is rounded up in a negative direction, is called min_Y', and the minimum value in the z-axis direction, whose decimal fraction is rounded up in a negative direction, is called min_Z'. Assume that orthogonal coordinates showing the position of any coarse-grained particle after rearrangement are represented by (X', Y,' Z'). The coarse-grained particle was rearranged at the position of (X'-min_X', Y'-min_Y', Z'-min_Z'). This operation was applied to all coarse-grained particles forming the target protein and all coarse-grained particles forming the peptide of interest, thereby further rearranging coarse-grained models of the target protein and the peptide of interest.

(Translation and rotational transfer of coarse-grained model)

Orthogonal coordinates showing the position of the i-th coarse-grained particle forming the target protein and the peptide of interest are represented by $(x_i, y_i, z_i)$. Assume that orthogonal coordinates showing the position of any coarse-grained particle are represented by $(X, Y, Z)$, and the total number of coarse-grained particles forming the target protein and coarse-grained particles forming the peptide of interest is N. The coarse-grained particle was arranged at a position shown by the following coordinates (9).
[Equation 23]

$$\left( X - \frac{\sum_i^N x_i}{N}, Y - \frac{\sum_i^N y_i}{N}, Z - \frac{\sum_i^N z_i}{N} \right) \ \cdots (9)$$

The coarse-grained particle arranged at the coordinates (9) was rotated counterclockwise around the x-axis by 3.142 rad, and counterclockwise rotated around the z-axis by 4.172 rad. Thereafter, the coarse-grained particle was translated in the y-axis direction by +1.267, and in the z-axis direction by +2.533. This operation was applied to all coarse-grained particles forming the target protein and all coarse-grained particles forming the peptide of interest. By this process, translation and rotational transfer of coarse-grained models were performed on each of the target protein and the peptide of interest.

Assume that orthogonal coordinates showing the position of any coarse-grained particle subjected to translation and rotational transfer are represented by $(X', Y,' Z')$. The coarse-grained particle was rearranged at the position of the following coordinates (10). This operation was applied to all coarse-grained particles forming the target protein and all coarse-grained particles forming the peptide of interest, thereby rearranging coarse-grained models of the target protein and the peptide of interest.
[Equation 24]

$$\left( X' + \frac{\sum_i^N x_i}{N}, Y' + \frac{\sum_i^N y_i}{N}, Z' + \frac{\sum_i^N z_i}{N} \right) \ \cdots (10)$$

(Generation of lattice structure)

A regular tetrahedral lattice was generated by setting 147 lattice points positioned on the following orthogonal coordinates.
(4,4,3), (5,5,4), (5,3,2), (3,5,2), (3,3,4), (4,6,5), (6,4,5), (6,6,3), (5,7,6), (3,7,4), (3,5,6), (7,5,6), (7,3,4), (5,3,6), (7,7,4), (7,5,2), (5,7,2), (4,2,1), (6,2,3), (6,4,1), (5,1,0), (3,3,0), (3,1,2), (7,1,2), (5,1,4), (7,3,0), (5,5,0), (2,4,1), (2,6,3), (4,6,1), (1,5,0), (1,3,2), (1,7,2), (1,5,4), (3,7,0), (2,2,3), (2,4,5), (4,2,5), (1,1,4), (1,3,6), (3,1,6), (4,8,7), (6,6,7), (6,8,5), (5,9,8), (3,9,6), (3,7,8), (7,7,8), (5,5,8), (7,9,6), (5,9,4), (2,8,5), (4,8,3), (1,9,4), (1,7,6), (3,9,2), (2,6,7), (4,4,7), (1,5,8), (3,3,8), (8,4,7), (8,6,5), (9,5,8), (9,3,6), (7,3,8), (9,7,6), (9,5,4), (8,2,5), (8,4,3), (9,1,4), (7,1,6), (9,3,2), (6,2,7), (5,1,8), (8,8,3), (9,9,4), (9,7,2), (7,9,2), (8,6,1), (9,5,0), (7,7,0), (6,8,1), (5,9,0), (4,0,-1), (6,0,1), (6,2,-1), (5,-1,-2), (3,1,-2), (3,-1,0), (7,-1,0), (5,-1,2), (7,1,-2), (5,3,-2), (2,2,-1), (4,4,-1), (1,3,-2), (1,1,0), (3,5,-2), (2,0,1), (4,0,3), (1,-1,2), (3,-1,4), (8,0,3), (8,2,1), (9,-1,2), (7,-1,4), (9,1,0), (6,0,5), (5,-1,6), (8,4,-1), (9,3,-2), (7,5,-2), (6,6,-1), (5,7,-2), (0,4,-1), (0,6,1), (2,6,-1), (-1,5,-2), (-1,3,0), (-1,7,0), (-1,5,2), (1,7,-2), (0,2,1), (0,4,3), (-1,1,2), (-1,3,4), (0,8,3), (2,8,1), (-1,9,2), (-1,7,4), (1,9,0), (0,6,5), (-1,5,6), (4,8,-1), (3,9,-2), (0,0,3), (0,2,5), (2,0,5), (-1,-1,4), (-1,1,6), (1,-1,6), (0,4,7), (2,2,7), (-1,3,8), (1,1,8), (4,0,7), (3,-1,8)

(Objective function)

The following equation (11) was used as an objective function for mathematical optimization.
[Equation 25]

$$E = E_{intra-molecular\ interaction} + E_{inter-molecular\ interaction} \ \cdots (11)$$

The first term of the right side represents the intramolecular interaction energy of the peptide of interest, and is given by the following equation (12).
[Equation 26]

$$E_{intra-molecular\ interaction} = \sum_{i=1}^{N-1} \sum_{j=i+1}^{N} a_{ij} e_{ij} \quad \dots (12)$$

The variable $a_{ij}$ is 1 when the shortest Euclidean distance between a coarse-grained particle belonging to the i-th amino acid residue of the peptide of interest and a coarse-grained particle belonging to the j-th amino acid residue of the peptide of interest is less than

[Equation 27]

$$6.5 \times \frac{\sqrt{3}}{3.8}$$

, and 0 when the shortest Euclidean distance is

[Equation 28]

$$6.5 \times \frac{\sqrt{3}}{3.8}$$

or more. The variable $e_{ij}$ is an energy value assigned to a pair of the type of the i-th amino acid residue of the peptide of interest and the type of the j-th amino acid residue of the peptide of interest in the Miyazawa-Jernigan matrix. N is the total number of coarse-grained particles forming the peptide of interest.

The second term of the right side of equation (11) represents the intermolecular interaction energy of the target protein and the peptide of interest, and is given by the following equation (13).
[Equation 29]

$$E_{inter-molecular\ interaction} = \sum_{i=1}^{N} \sum_{j=1}^{L} b_{ij} e_{ij} \quad \dots (13)$$

The variable $b_{ij}$ is 1 when the shortest Euclidean distance between a coarse-grained particle belonging to the i-th amino acid residue of the peptide of interest and a coarse-grained particle belonging to the j-th amino acid residue of the target protein is less than

[Equation 30]

$$6.5 \times \frac{\sqrt{3}}{3.8}$$

, and 0 when the shortest Euclidean distance is

[Equation 31]

$$6.5 \times \frac{\sqrt{3}}{3.8}$$

or more. The variable $e_{ij}$ is an energy value assigned to a pair of the type of the i-th amino acid residue and the type of the j-th amino acid residue of the peptide of interest in the Miyazawa-Jernigan matrix. N is the total number of coarse-grained particles forming the peptide of interest. L is the total number of coarse-grained particles forming the target protein.

(Constraint condition)

The following four constraint conditions were set in calculation for mathematical optimization.

(1) All coarse-grained particles forming the peptide of interest must be placed at lattice points of a regular tetrahedral lattice, and a plurality of coarse-grained particles must not be placed at one lattice point.

(2) A coarse-grained particle corresponding to the i-th amino acid residue of the peptide of interest and a coarse-grained particle corresponding to the (i+1)-th amino acid residue of the peptide of interest must be placed at two lattice points adjacent to each other.

(3) A coarse-grained particle corresponding to Pro1 and a coarse-grained particle corresponding to the main chain region of Gly6 in the peptide of interest must be placed at two lattice points adjacent to each other.

(4) A coarse-grained particle of the peptide of interest must not be placed at a lattice point positioned such that the Euclidean distance between the lattice point and a coarse-grained particle forming the target protein is within the following value.

[Equation 32]

$$3 \times \frac{\sqrt{3}}{3.8}$$

(Mathematical optimization)

For performing mathematical optimization including calculation of interaction energy, OR-Tools version 9.5.2237(https://pypi.org/project/ortools/9.5.2237/), one of constraint programming methods, was used. Or-Tools was used to input a coarse-grained model in which the target protein was rearranged by translation and rotational transfer. An arrangement pattern for coarse-grained particles forming the peptide of interest, which gives the minimum value of the objective function E subject to the constraint condition was obtained as a docking model. The minimum value of the objective function E here was -72.51.

(Verification)

the consistency of the obtained docking model with a model obtained by coarse-graining of C-chain of the crystal structure "3WNE" was verified. As an index of the verification, a realscale root mean square deviation (RMSD) represented by the following equation (14) was calculated.
[Equation 33]

$$RMSD = \frac{3.8}{\sqrt{3}} \times \frac{1}{N} \sqrt{\sum_{i}^{N} d_i^2} \quad \ldots (14)$$

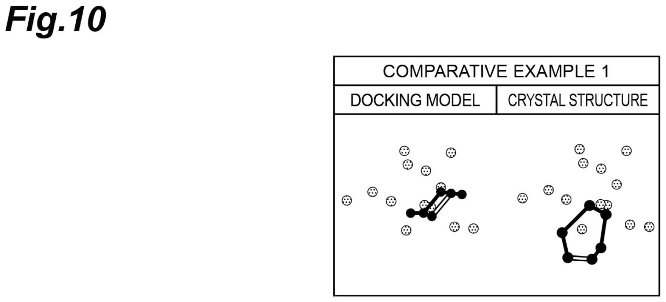

Here, the variable $d_i$ represents a Euclidean distance between the position of the i-th coarse-grained particle in the docking model and the position of the i-th coarse-grained particle in the coarse-grained model of the peptide of interest after rearrangement by translation and the rotating operation. N is the total number of coarse-grained particles forming the peptide of interest. As a result, it was confirmed that a docking model with a root mean square deviation of 8.25 Å had been obtained from the coarse-grained of the peptide of interest after rearrangement by translation and the rotating operation. The docking model and the rearranged crystal structure in Comparative Example 1 are shown in Figure 10. The shaded circle denotes a coarse-grained particle of the target protein. The black circle denotes a coarse-grained particle corresponding to an amino acid residue of the peptide of interest. It was found that the docking model and the crystal structure deviate from each other when an amino acid residue was approximated by one coarse-grained particle as shown in Figure 10.

[Reference Signs List]

10...estimation system, 11...acquisition unit, 12...generation unit, 13...calculation unit, 14...estimation unit, 110...estimation program, 200...target molecule model, 201...coarse-grained particle, 210...center of gravity, 220...lattice system, 310, 320...constraint condition, 311, 321...intermolecular constraint, 312, 322...intramolecular constraint, 313, 323...cyclization constraint, 230, 240, 250, 260, 270, 280...molecule-of-interest model, 231, 241, 251, 261, 262, 271, 272, 281, 282...coarse-grained particle.

**Claims**

1.  An estimation system comprising at least one processor,
    wherein the at least one processor

    acquires a target molecule model representing a conformation of a target molecule,
    generates a molecule-of-interest model representing a conformation of a molecule of interest, the molecule of interest being a molecule capable of binding to the target molecule,
    calculates energy of interaction between the molecule of interest and the target molecule as intermolecular interaction energy based on a positional relationship between the target molecule model and the molecule-of-interest model,
    calculates energy of interaction in the molecule of interest as intramolecular interaction energy, and
    estimates a conformation of the molecule of interest based on the intermolecular interaction energy and the intramolecular interaction energy.

2.  The estimation system according to claim 1,
    wherein the at least one processor

    calculates a plurality of energies of interaction between a plurality of constituent units forming the target molecule and a plurality of constituent units forming the molecule of interest, as a plurality of energies of interaction between units, and
    calculates the intermolecular interaction energy based on the plurality of energies of interaction between units.

3.  The estimation system according to claim 1 or 2,
    wherein the at least one processor

    calculates a plurality of energies of interaction between a plurality of constituent units forming the molecule of interest, as a plurality of energies of interaction between units, and
    calculates the intramolecular interaction energy based on the plurality of energies of interaction between units.

4.  The estimation system according to claim 2 or 3,
    wherein the at least one processor

    acquires the target molecule model representing the plurality of constituent units forming the target molecule by coarse-grained particles, and
    generates the molecule-of-interest model representing the plurality of constituent units forming the molecule of interest by the coarse-grained particles.

5.  The estimation system according to claim 4,
    wherein the at least one processor

    arranges a j-th constituent unit and a (j+1)-th constituent unit forming the molecule of interest such that the constituent units are adjacent to each other, with j being an integer of 1 or more, and
    generates the molecule-of-interest model representing the j-th constituent unit and the (j+1)-th constituent unit by the coarse-grained particles.

6.  The estimation system according to claim 4 or 5,
    wherein the at least one processor generates the molecule-of-interest model representing at least one of the plurality of constituent units forming the molecule of interest by a plurality of the coarse-grained particles.

7.  The estimation system according to claim 6,
    wherein the at least one processor generates the molecule-of-interest model representing at least one of the plurality of constituent units forming the molecule of interest by two of the coarse-grained particles.

8.  The estimation system according to any one of claims 4 to 7,
    wherein the at least one processor generates the molecule-of-interest model representing at least one of the plurality of constituent units forming the molecule of interest by the coarse-grained particle representing a main chain and the coarse-grained particle representing a side chain.

9. The estimation system according to claim 8,
wherein the at least one processor generates the molecule-of-interest model by arranging the coarse-grained particle representing the main chain and the coarse-grained particle representing the side chain such that the coarse-grained particles are adjacent to each other.

10. The estimation system according to any one of claims 1 to 9,
wherein the at least one processor

generates the molecule-of-interest model for each of the plurality of conformations of the molecule of interest,
calculates the intermolecular interaction energy based on the positional relationship between the target molecule model and the generated molecule-of-interest model, and
selects at least one conformation from the plurality of conformations of the molecule of interest based on the intermolecular interaction energy of each of the plurality of conformations of the molecule of interest.

11. The estimation system according to claim 10,
wherein the at least one processor

generates the molecule-of-interest model for each of the plurality of conformations of the molecule of interest,
calculates energy of interaction in the molecule of interest as intramolecular interaction energy based on the generated molecule-of-interest model, calculates a sum of the intramolecular interaction energy and the intermolecular interaction energy as total interaction energy, and
selects the at least one conformation from the plurality of conformations of the molecule of interest based on the total interaction energy of each of the plurality of conformations of the molecule of interest.

12. The estimation system according to any one of claims 1 to 11,
wherein the at least one processor generates the molecule-of-interest model so as to satisfy a constraint condition including an intermolecular constraint about the positional relationship between the molecule-of-interest model and the target molecule model.

13. The estimation system according to claim 12,
wherein the intermolecular constraint indicates that steric hindrance is avoided between the molecule of interest and the target molecule.

14. The estimation system according to claim 12 or 13,
wherein the at least one processor

sets an outer edge of a lattice system for arranging the conformation of the molecule of interest, based on the target molecule model, and
generates the molecule-of-interest model in the lattice system such that the molecule-of-interest model satisfies the constraint condition in the lattice system.

15. The estimation system according to claim 14,
wherein the at least one processor

acquires the target molecule model representing the conformation of an active site in the target molecule, and
sets the outer edge of the lattice system based on a center of gravity of a plurality of constituent units forming the active site.

16. The estimation system according to claim 14 or 15,
wherein a unit cell of the lattice system is one selected from a regular tetrahedral lattice, a cubic lattice and a square lattice.

17. The estimation system according to any one of claims 14 to 16,
wherein the at least one processor generates the molecule-of-interest model in the lattice system by arranging two coarse-grained particles corresponding to a j-th constituent unit and a (j+1)-th constituent unit forming the molecule of interest such that the coarse-grained particles are adjacent to each other in the lattice system, with j being an integer of 1 or more.

**18.** The estimation system according to any one of claims 14 to 17,
wherein the at least one processor generates the molecule-of-interest model in the lattice system by arranging a coarse-grained particle representing a main chain and a coarse-grained particle representing a side chain such that the coarse-grained particles are adjacent to each other for at least one of a plurality of constituent units forming the molecule of interest in the lattice system.

**19.** The estimation system according to any one of claims 12 to 18,
wherein the constraint condition further includes at least one of an intramolecular constraint indicating that steric hindrance is avoided between a plurality of constituent units forming the molecule of interest and a cyclization constraint indicating that the molecule of interest contains a cyclic structure.

**20.** The estimation system according to any one of claims 1 to 19,

wherein the target molecule is a protein, and
the molecule of interest is a peptide.

**21.** A method for estimating a molecular conformation, which is executed by an estimation system comprising at least one processor, the method comprising the steps of:

acquiring a target molecule model representing a conformation of a target molecule;
generating a molecule-of-interest model representing a conformation of a molecule of interest, the molecule of interest being a molecule capable of binding to the target molecule;
calculating energy of interaction between the molecule of interest and the target molecule as intermolecular interaction energy based on a positional relationship between the target molecule model and the molecule-of-interest model;
calculating energy of interaction in the molecule of interest as intramolecular interaction energy; and
estimating a conformation of the molecule of interest based on the intermolecular interaction energy and the intramolecular interaction energy.

**22.** An estimation program that causes a computer to execute the steps of:

acquiring a target molecule model representing a conformation of a target molecule;
generating a molecule-of-interest model representing a conformation of a molecule of interest, the molecule of interest being a molecule capable of binding to the target molecule;
calculating energy of interaction between the molecule of interest and the target molecule as intermolecular interaction energy based on a positional relationship between the target molecule model and the molecule-of-interest model;
calculating energy of interaction in the molecule of interest as intramolecular interaction energy; and
estimating a conformation of the molecule of interest based on the intermolecular interaction energy and the intramolecular interaction energy.

## Fig.1

ESTIMATION SYSTEM — 10

ACQUISITION UNIT — 11

GENERATION UNIT — 12

CALCULATION UNIT — 13

ESTIMATION UNIT — 14

# *Fig.2*

# *Fig.3*

S1

START

S11
ACQUIRE TARGET MOLECULE MODEL

S12
ACQUIRE MOLECULE-OF-INTEREST DATA

S13
SET LATTICE SYSTEM BASED ON
TARGET MOLECULE MODEL

S14
GENERATE MOLECULE-OF-INTEREST MODEL
IN LATTICE SYSTEM SO AS
TO SATISFY CONSTRAINT CONDITION

S15
CALCULATE INTERACTION ENERGY
BASED ON TARGET MOLECULE MODEL
AND MOLECULE-OF-INTEREST MODEL

S16
FINISH SEARCH?

NO

YES

S17
ESTIMATE CONFORMATION OF
MOLECULE OF INTEREST

S18
OUTPUT ESTIMATION RESULT

END

*Fig.4*

# *Fig.5*

**Fig.6**

## Fig.7

| EXAMPLE 1 | | EXAMPLE 2 | |
|---|---|---|---|
| DOCKING MODEL | CRYSTAL STRUCTURE | DOCKING MODEL | CRYSTAL STRUCTURE |

| EXAMPLE 3 | | EXAMPLE 4 | |
|---|---|---|---|
| DOCKING MODEL | CRYSTAL STRUCTURE | DOCKING MODEL | CRYSTAL STRUCTURE |

EP 4 769 418 A1

Fig.8

*Fig.9*

EP 4 769 418 A1

*Fig.10*

| COMPARATIVE EXAMPLE 1 | |
|---|---|
| DOCKING MODEL | CRYSTAL STRUCTURE |

## INTERNATIONAL SEARCH REPORT

| International application No. |
| --- |
| **PCT/JP2024/038563** |

| A. | CLASSIFICATION OF SUBJECT MATTER |
| --- | --- |

*G16C 10/00*(2019.01)i; *G16C 20/00*(2019.01)i
FI:   G16C10/00; G16C20/00

According to International Patent Classification (IPC) or to both national classification and IPC

| B. | FIELDS SEARCHED |
| --- | --- |

Minimum documentation searched (classification system followed by classification symbols)

G06Q10/00-99/00; G16B5/00-99/00; G16C10/00-99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 93/20525 A1 (ITAI, Akiko) 14 October 1993 (1993-10-14) p. 3, lower right column, line 19, p. 4, lower left column, line 27, p. 5, upper left column, line 1 - lower left column, line 29, p. 6, upper left column, line 8 - p. 7, lower left column, line 7 | 1-7, 10-14, 16-17, 19-22 |
| A | entire text, all drawings | 8-9, 15, 18 |
| Y | 根上 樹, 粗視化分子動力学シミュレーションを用いたタンパク質-リガンド結合過程の網羅的解析, [online], The University of Tokyo, 10 August 2016, pp. 1-19, [retrieved on 20 November 2024], Internet: <URL: https://repository.dl.itc.u-tokyo.ac.jp/record/8342/files/A31590.pdf>, <DOI: 10.15083/00008333>, non-official translation (NEGAMI, Tatsuki, Comprehensive analysis of protein-ligand binding processes using coarse-grained molecular dynamics simulations) p. 4, line 13, pp. 17-19, "3.2.2 Coarse-grained model mapping and parameters" | 1-7, 10-14, 16-17, 19-22 |
| A | entire text, all drawings | 8-9, 15, 18 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 November 2024** | **03 December 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/038563**

| Patent document cited in search report | Publication date (day/month/year) | Patent family member(s) | Publication date (day/month/year) |
|---|---|---|---|
| WO 93/20525 A1 | 14 October 1993 | US 5642292 A column 3, lines 21-23, column 5, line 66 - column 6, line 2, column 6, line 66 - column 9, line 15, column 9, line 23 - column 13, line 54 EP 633534 A1 p. 4, lines 34-35, p. 6, lines 24-26, p. 7, line 29 - p. 9, line 16, p. 9, line 25 - p. 12, line 48 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020091518 A
- JP 2020173643 A
- JP 2020194487 A

**Non-patent literature cited in the description**

- **ROBERT, A.** ; **BARKOUTSOS, P.K.** ; **WOERNER, S.** ; **TAVERNELLI, I.** Resource-efficient quantum algorithm for protein folding. *npj Quantum Inf.*, 2021, vol. 7, 38
- *J. Mol. Biol.*, 1996, vol. 256, 623-644